# EUROPEAN PATENT APPLICATION

(11) **EP 2 189 538 A1**
(43) Date of publication of application: **26.05.2010**
(21) Application number: 08169662.7
(22) Date of filing: 21.11.2008
(51) Int. Cl.: C12Q 1/68

(54) **An analysis molecule and method for the analysis of a nucleotide position in a nucleic acid**

(71) Applicant: Epigenomics AG, 10178 Berlin (DE)
(72) Inventor: Tetzner, Reimo, 10439 Berlin (DE)

(57) **Abstract**

The invention relates to an analysis molecule for the analysis of one or more nucleotide positions in a nucleic acid. Said positions are in a first state or an alternative second state. The invention further relates to inventive methods that comprise the use of the inventive analysis molecule. The invention further relates to inventive kits that comprise the inventive analysis molecule.

## Description

The invention relates to an analysis molecule, a method and a kit for the analysis of a nucleotide position in a nucleic acid.

Throughout this application, various publications are cited. The disclosure of these publications is hereby incorporated by reference in its entirety into this application to describe more fully the state of the art to which this invention pertains.

It has long been known in the art that alterations of single positions within the sequence of nucleic acids can have an effect on function of the encoded protein, alter regulatory mechanisms and cause disease. It is therefore necessary to analyze such alterations.

Known analysis methods are often performed on the basis of nucleic acid amplification reactions, such as but not limited to polymerase chain reaction (PCR), ligase chain reaction (LCR) or primer extension.

A problem arises from the fact the nucleic acid to be analyzed is usually present together with other nucleic acids that may differ from the nucleic acid of interest only at a single position or at a few positions, which makes it difficult to distinguish between the different nucleic acid species.

One approach described in the art to circumvent this problem has been the use of blocking molecules (or blockers). Such a blocking molecule is specific for one species of the nucleic acid and hybridizes only with the nucleic acid of that particular species, while it does not hybridize to a sequence that differs at the position in question. The presence of the blocker on the template nucleic acid prevents the polymerase from amplifying the nucleic acid that the blocker hybridizes with. Therefore, only the nucleic acid with a particular sequence is amplified, whereas the amplification of similar sequences is inhibited through binding of the blocker. Thus, a selective amplification of only one species of the nucleic acid can be performed.

Accordingly, in the amplification reaction, primers as well as a blocker need to be present. One disadvantage attached to this approach is that for template-specific primers of about 18 to 22 bases, the blocker needs be of about 30 - 40 bases in length in order to achieve a Tₘ value (Tₘ: DNA melting temperature, the temperature at which half of the given DNA strands are in the double-helical state, and the other half are in random-coil states) that makes a successful species-specific amplification of the nucleic acid to be analyzed possible. At the same time, such a length, however, is of a disadvantage when analyzing a single or only a few polymorphic positions in a nucleic acid. Therefore, it has thus far not been possible to analyze a number of less than three or four positions simultaneously with this approach.

In the prior art, methods have been described that use a blocker that consists of a "locked nucleic acid" (LNA) or a "peptide nucleic acid" (PNA) (e.g. Orum et al., Single base pair mutation analysis by PNA directed PCR clamping. Nucleic Acids Res. 1993; 21(23): 5332-6). Compared to an unmodified polynucleotide blocker molecule such blockers have an increased Tₘ value for a given length. In spite of that, no such blocker has been described that allows for the species-specific detection of a nucleotide position that is present within a pool of nucleic acid molecules of an alternative species in a frequency of less than 1 %. Therefore, the blocker molecules known so far do not exhibit a sufficient sensitivity, in particular for the species-specific analysis of a few nucleotide positions or even a single nucleotide position in a nucleic acid present within a pool of an alternative species of the nucleic acid.

### Description of the invention

Accordingly, the problem underlying the present invention was to provide a means of analyzing a single nucleotide position or analyzing a few nucleotide positions ( in particular 2, 3, 4, 5, 6, 7 or more) located close to each other. In particular, detection of such a nucleotide position was to be enabled in spite of a large abundance of molecules of a similar sequence.

An analysis molecule according to the present invention surprisingly solves this problem. Such an analysis molecule can be used for the detection of a single nucleotide polymorphism (SNP) or mutation as well as for the detection of the methylation status of a cytosine residue in a nucleic acid. Generally, the nucleotide position can be present in a first state or an alternative second state. In the case of an SNP, the wild type (first) state can be distinguished from a mutant (second) state. In the case of methylation, both a methylated cytosine residue (first state) and an unmethylated cytosine residue (second state) can be analyzed.

According to the invention, the analysis molecule comprises a first and a second region. Said first region serves as a primer by hybridizing with the nucleic acid to be analyzed such as but not limited to a template DNA. Said second region serves as a blocker being specific for a state not of interest. Said state may either be the first state or an alternative second state. Amplification performed with the inventive analysis molecule proceeds only for one template species, i.e. the nucleic acid molecules present in the state of interest (either the second or the first state). If the nucleotide in question is present in a state not of interest, the blocker acting part of the inventive analysis molecule binds to the extension product initiated by the primer acting part of said analysis molecule. This has the consequence that the generation of further amplification products is suppressed or even no further amplification product is generated.

To allow initiation of an amplification reaction, the first region of the analysis molecule is located on the 3' side and the second region is located on the 5' side of the molecule.

The present invention allows the use of a shorter blocking sequences than described in the state of the art when analyzing nucleotide positions. By attaching the blocking sequence to the primer sequence, the hybridization of the blocking part to the amplification template becomes an intramolecular process. Compared to the hybridization of two independent molecules, the Tₘ value of the oligonucleotides is increased, because the molecules are sterically brought into each other's vicinity. The blocking sequence of the invention can therefore be shorter than described thus far, which leads to more efficient discrimination of the target nucleotide to be analyzed. In addition, no excess of free blocker over the generated amplified molecules needs to be present in the amplification reaction, as is the case when primer and blocker sequences are encoded by separate molecules.

In addition, the use of an inventive analysis molecule in view of a conventional blocker has the advantage that one compound is less necessary for specific amplification. Thus, the number of controls and quality checks is reduced and therewith also costs and efforts. This is in particular of importance wherein a large number of samples is processed such as but not limited to in the discovery of new markers / methylation markers, and in the diagnostic tests of patient samples. Furthermore, further costs and efforts are saved because the inventive analysis molecule has less modifications and is in average much shorter than corresponding conventional pairs of blocker and primer.

In a preferred embodiment of the invention the first region of the analysis molecule is separated from the second region of the analysis molecule by a third region. In a preferred embodiment, said third region has a hinge-like function and enables the second region to sterically move freely with respect to the first region. This enables the blocker to fold back and form a loop. In a preferred further embodiment, said third region stops a polymerase and leads to the dissociation of the amplification complex. Therefore, the analysis molecule is not amplified completely during an amplification reaction. In particular preferred embodiment, said third region enables a sterical free movement and stops polymerization.

It is preferred that the first region of the analysis molecule hybridizes with a first sequence portion of the nucleic acid to be analyzed. In one embodiment, the primer part of the analysis molecule binds to the template without distinguishing between a first state and an alternative second state of the nucleotide position of interest. In an alternative embodiment, the primer part of the analysis molecule binds to the template and does distinguish between a first state and an alternative second state of the nucleotide position of interest. This leads to a further increase in specificity of detection between different species of template nucleic acid molecules. However, regardless of which kind of primer acting part of the analysis molecule is chosen, after extension of the primer acting part, the second region of the analysis molecule (blocker acting part) hybridizes intramolecularly to the region of the extension product that contains the nucleotide position to be analyzed. This amplification product with the nucleotide of interest is generated in the amplification reaction that is primed with the first region of the analysis molecule.

In a preferred embodiment, the sequence portion of the extension product to which the second region of the analysis molecule hybridizes intramolecularly, overlaps at least partially with the sequence portion onto which a second primer hybridizes. Said second primer serves as a starter for the amplification of the reverse complementary region. Through this overlap of preferably one to fifteen bases, more preferably of one, two, three, four, five, six, seven, eight, nine or ten bases, and most preferably of two, three, four or five bases, the second primer can only bind to the template and initiate an amplification reaction if the blocker acting part of the analysis molecule is not bound due to sequence mismatches.

In a preferred embodiment, the second region of the analysis molecule comprises one or more modifications that render the second region non-hydrolysable. Therefore, the hybridizing region of the second region does not need an overlap with the hybridizing region of said second primer. The amplification is still suppressed by the back-folded hybridized blocker acting part. The reason for this is, the polymerase is not able to hydrolyze i.e. to displace or remove the second region of the analysis molecule (blocker acting part) from the template nucleic acid.

In a preferred embodiment, the first region, the second region , or both regions of the analysis molecule comprises no modifications. In a particular preferred embodiment, the inventive analysis molecule consisting only of a first region (primer acting part) and of a second region (blocker acting part) comprises no modifications. The use of analysis molecules without 5' modifications is in particular be preferred for the amplification by means of enzymes lacking 5' exonuclease activity. Preferably, the hybridizing region of the second region (blocker acting part) overlaps with the hybridizing region of the second primer used for amplification.

According to a preferred embodiment, the first region, and/or the second region, and/or the third region of the analysis molecule is an oligonucleotide. Preferably an analysis molecule comprises a DNA, PNA, RNA, GNA (glycerol nucleic acid), TNA (threose nucleic acid), LNA (locked nucleic acid), a gripNA, or a O-MeRNA nucleotide or combinations thereof. Preferably, the third region of the analysis molecule is an alkylic group, a PNA monomer, an abasic site or another chemical functional group that enable the covalent link between the first and second region or combinations thereof. Preferably, the alkylic group has more than six carbon atoms to provide for the necessary flexibility. In particularly preferred is a C18 spacer group (HEG, hexaethylenglycol). However it is also possible and herewith preferred to use cross linked bases, backbone modifications or base modifications at a 3' terminal nucleotide or monomer of the blocker acting part of the analysis molecule. Preferably, due to this, no 3' modification of the blocker acting part (second region of the analysis molecule) is needed, since the polymerase cannot read over the third region of the analysis molecule and will not degrade the blocker acting part from its 3' side. Thus 3' modifications of the blocker acting sequence are dispensable. This saves costs for providing such a modification and efforts for its quality control.
Preferably, between the first, second and/or third region of the analysis molecule, an additional sequence or linker can be present.

Preferably, the analysis molecule according to the present invention can, for example, be used for the detection of a single nucleotide polymorphism (SNP) in a nucleic acid. The wild type (first) state can be distinguished from a mutant (second) state independent of which mutation is present. If, for example, but not limited to, the wild type allele is A, the mutation allele (either C, G, or T) can be analyzed by blocking the amplification of the wild type nucleic acid. For this the blocker acting part of the analysis molecules contains an A at said position to be able to hybridize with the extension product initiated by the first region (primer acting part) of said analysis molecule. Said wild type allele A in the template nucleic acid leads to a T in the extension product initiated by the first region of the analysis molecule (reverse complementary strand). The blocker acting part of said analysis molecule containing an A at said position therefore binds to said T and prevents further amplification.

Gene regulation has been correlated with methylation of a gene or genome. Certain cell types consistently display specific methylation patterns. This has been shown for a number of different cell types (Adorjan et al. (2002) Tumor class prediction and discovery by micro array-based DNA methylation analysis. Nucleic Acids Res 30(5), e21).

In vertebrates, DNA is methylated nearly exclusively at cytosine bases located 5' to guanine. Such sites are being referred to as CpG dinucleotides. This modification has important regulatory effects on gene expression, especially when involving CpG rich areas, known as CpG islands, located in the promoter regions of many genes. While almost all gene-associated islands are protected from methylation on autosomal chromosomes, extensive methylation of CpG islands has been associated with transcriptional inactivation of selected imprinted genes and genes on the inactive X-chromosome of females.

Differential methylation patterns have great relevance for understanding disease and developing diagnostic applications. The identification of 5-methylcytosine within a DNA sequence is of importance in order to uncover its role in gene regulation. The position of a 5-methylcytosine cannot be identified using methods that are based on base pair behavior, since methylated cytosine behaves just as an unmethylated cytosine as per its hybridization preference.

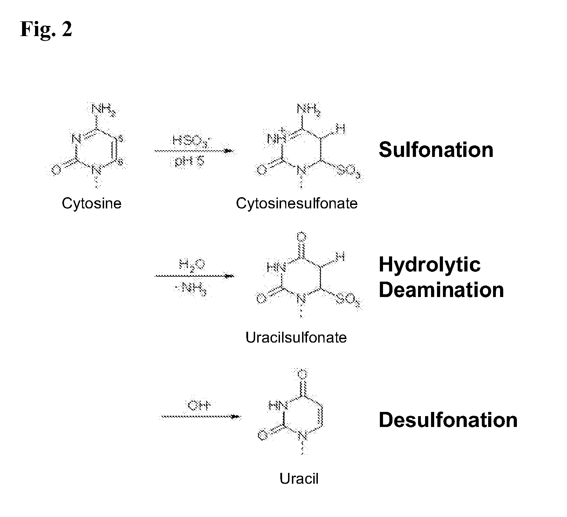

Since this epigenetic methylation information will be lost in any standard amplification, the nucleic acid is usually treated with bisulfite prior to analysis, as will be explained below (see also figure 2 and the description thereto). Alternatively, it is possible to convert the nucleic acid enzymatically, e.g. with a cytidine deaminase. Such an enzyme is described in Bransteitter et al.: Activation-induced cytidine deaminase deaminates deoxycytidine on single-stranded DNA but requires the action of RNase. Proc Natl Acad Sci U S A 2003; 100(7): 4102-4107; WO 2005/005660.

In case the analysis molecule according to the invention is to be used to analyze the methylation state of cytosine residues, particularly in genomic DNA, the blocker acting part has to contain residues that allow the distinction between methylated and non-methylated DNA.

After bisulfite treatment, the two strands of a double stranded DNA molecule lose their complementation. Both of them contain low quantities of cytosine bases, since all non-methylated cytosines have been converted to uracils. For this reason, these two strands are referred to as "G-rich strands" (guanines are more abundant in comparison to cytosines). In contrast to native DNA, these two strands are herein also called "bisulfite sense strands". Both of these bisulfite sense strands may act as a template DNA for analyzing the same CpG sites. Accordingly, the strands which are generated during an amplification reaction, and which are complementary to these bisulfite sense strands, are herein referred to as "bisulfite anti-sense strands". Since bisulfite anti-sense strands are complementary to the bisulfite sense strands, they contain low quantities of guanines and are therefore also called "C-rich strands".

According to the invention, the inventive analysis molecule is used to analyze the methylation of one, two, three, four, five, six, seven or more defined CpG positions. In a first step, the nucleic acid to be analyzed is treated with bisulfite or a cytidine-deaminase. In a second step, methylation of the original nucleic acid is detected by applying the inventive analysis molecule to the treated nucleic acid. In a particular preferred embodiment, the analysis molecule comprises a C-rich first region acting as a primer and a G-rich second region acting as a blocker. If the case may be, analysis of a methylated site takes place by blocking the amplification of treated nucleic acids resembling the unmethylated state. For this, the second region contains a thymine residue at the position that corresponds to the unmethylated state. In order to analyze the unmethylated site, amplification of treated nucleic acids resembling the methylated state are blocked. For this, the second region acting as a blocker contains a cytidine at the position to be analyzed.

In a particular preferred embodiment, the analysis molecule comprises a G-rich first region acting as a primer and a C-rich second region acting as a blocker. If the case may be, analysis of a methylated site takes place by blocking the amplification of treated nucleic acids resembling the unmethylated state. For this, the second region contains an adenine residue at the position that corresponds to the unmethylated state. In order to analyze the unmethylated site, amplification of treated nucleic acids resembling the methylated state are blocked. For this, the second region acting as a blocker contains a guanine residue at the position to be analyzed.

In effect, the blocker allows the amplification of only one nucleic acid species, either the one corresponding to the methylated state or the one corresponding to the unmethylated state of the CpG position in question.

According to particular preferred embodiment, two or more (preferably but not limited to 3, 4, 5, 6, or 7) CpG positions are simultaneously analyzed. For this, the part of the inventive analysis molecule acting as a blocker comprises all 2, 3, 4, 5, 6, 7 or more CpG positions. Each of the said positions may be present either in the methylated or unmethylated state. Thus, it is possible to analyze a multiplicity of CpG positions by means of the inventive analysis molecule wherein all of said positions are present in the methylated state, wherein all of the said positions are present in the unmethylated state, or wherein parts of the said positions are present in the methylated state while the other part of said positions are present in the unmethylated state.

When analyzing for the presence of a particular nucleic acid species in a pool of nucleic acids containing also a nucleic acid species that differs from the species to be analyzed only in one or a few nucleotide positions, a specific amplification of only the nucleic acid species in question is made possible, even over a "background" of an alternative species that is present in a 1000fold excess over the species to be analyzed. Incorrect results caused by amplification of templates of the other species are prevented.
The underlying problem of the invention is also solved by a method for the analysis of a nucleotide position, such as a single nucleotide polymorphism (SNP) or a cytosine residue that is either methylated or unmethylated in a nucleic acid.

This method comprises the steps of a) providing an analysis molecule as described above, and b) amplifying the nucleic acid to be analyzed in the presence of the analysis molecule.

Using this method it is possible to analyze the presence and the amount of a first nucleic acid species (with a particular sequence) in the presence of a second nucleic acid species that differs from the first nucleic acid species only in one nucleotide or a few (preferably in two, three, four, five, six, seven or more) nucleotides located close to each other. Cross-interference of the nucleic acid species that is not of interest and incorrect results are thereby avoided.

According to the invention, the amplification is preferably a polymerase chain reaction (PCR), a ligase chain reaction (LCR), a primer extension(???) , or a combination of LCR and primer extension. In particular, it is preferred that the amplification method is PCR. In particular, it is preferred that the polymerase is a heat stable polymerase, such as but not limited to Taq polymerase or Pfu polymerase. In particular, it is preferred that the ligase is a heat stable ligase.

In case the amplification comprises LCR, it is particularly preferred, that the 5' terminal nucleotide of the blocker acting part so modified to prevent ligation of said nucleotide.

In case the amplification comprises LCR and primer extension, it is preferred in an embodiment, that, in each cycle, the primer extension reaction is carried out before the subsequent corresponding ligation.

In case the amplification is primer extension, it is preferred in an embodiment that the primer extension is carried out in two steps. In the first step, a primer extension template is generated by means of inventive analysis molecule. In the second step, the actual primer extension by means of a primer to be extended and by means of a polymerase is performed. Preferably, in the first step, the inventive analysis molecule binds sequence specifically to the nucleic acid to be amplified. Subsequently, the primer acting part (first region) of the analysis molecule is extended followed by the removal of the not extended analysis molecules. Preferably, in the second step, the extended primer acting part (that is the template) and a primer to be extended are subject to a primer extension reaction. Wherein the blocker acting part is able to hybridize intramolecularly with at least parts of the synthesized part of the extended analysis molecule, the primer binding is prohibited, said primer also being able to hybridize onto at least parts of the synthesized part of the extended analysis molecule. Wherein the blocker acting part is unable to hybridize intramolecularly with at least parts of the synthesized part of the extended analysis molecule, the primer binding onto at least parts of the synthesized part take place. Thereafter, the bound primer is ready to be extended.

It is preferred that the method according to the invention is performed with a second primer to allow for performing a polymerase chain reaction (PCR). In one embodiment of the invention, the second primer, the first primer, or both does not distinguish between a first state and an alternative second state of the nucleic acid but rather allows unbiased amplification. In an alternative embodiment, the primer and/or the inventive analysis molecule are configured to distinguish the template nucleic acid to be analyzed in at least one position. Preferably, one or more of said at least one position is located at the 3' end of the second primer and/or at the 3' end of the first region of the analysis molecule.

Preferably, the primer acting part of the analysis molecule is used to detect mutations and/or SNPs. Correspondingly, the inventive analysis molecule is subjected to known methods of mutation, SNP or genotype analysis. For example but not limited to such methods are the ARMS method (Newton et al., 1989, Analysis of any point mutation in DNA. The amplification refractory mutation system (ARMS). Nucleic Acids Res., 17:, 2503-2516) or tetra primer PCR method (Ye S. et al., 1992, Allele specific amplification by tetra-primer PCR. Nucleic Acids Res., 20:, 1152).

In addition, preferably, the primer acting part of the analysis molecule is used to detect methylation. Correspondingly, the inventive analysis molecule is subjected to known methods of methylation analysis. For example but not limited to such a method is methylation specific PCR (MSP; Herman et al.: Methylation-specific PCR: a novel PCR assay for methylation status of CpG islands. Proc Natl Acad Sci U S A. 1996 Sep 3; 93(18): 9821-6).

In a particular preferred embodiment of the method, the second primer used is an analysis molecule as described above in addition to the analysis molecule used as first primer. This has the advantage that the blocking performed will be more specific and the analysis performed will be more sensitive. The reason for this is that two blocking sequences (the blocker acting part of the first analysis molecule and the blocker acting part of the second analysis molecule) will be used to suppress the amplification of undesired nucleic acid species. Due to this increased efficiency in blocking the unwanted amplification, the use of two analysis molecules allows to detect and/or quantify lower amounts of the desired nucleic acid species in comparison to the use of just one analysis molecule or the use of a conventional blocker molecule. Thus, said embodiment of two analysis molecules used for amplification is characterized by an increased sensitivity and is herewith particularly preferred.

In a preferred embodiment, the quantification or detection of amplified molecules using the described analysis molecule takes place during PCR amplification.

In order to quantify the amount of molecules of a particular nucleic acid species, the amplification or the nucleic acid is performed preferably in the presence of a labeled probe. This labeled probe generates a detectable signal upon sequence specific binding to the amplification product. According to preferred embodiment, amplification products are detected and/or quantified by means of intercalating dyes such as but not limited to SYBR® green. The use of a labeled probe or the use of intercalating dyes also allows performing the present method as a real time polymerase chain reaction. According to a preferred embodiment, amplification is performed by use of a labeled primer.

Preferably the probe used in a real time polymerase chain reaction can be any probe that is known in the state of the art, such as FRET probes (LightCycler, TaqMan, molecular beacon, Scorpion primer, Pleiades probes, displacing probes, Light-up probes or Lux probes). The use of Ethidiumbromide, SYBR® green, SYTO-dyes or other nucleic acid intercalating dyes is also possible in embodiments of the method of the invention.

To quantitate the measurements, the signal from the probe or the used intercalated dye is measured. In real time PCR analysis, the Ct (threshold point) or the Cp value (crossing point) is determined. In order to obtain absolute values, a standard should be used with the method, either as an external or internal standard.

To analyze the methylation status of a nucleic acid, it is necessary to make methylated cytosines distinguishable form non-methylated cytosines. This is done according to the present invention prior to amplifying the nucleic acid by treating it with a chemical reagent or an enzyme, whereby the base pairing behavior of methylated cytosine bases and/or unmethylated cytosine bases of the nucleic acid are altered such that methylated cytosine bases become distinguishable from unmethylated cytosine bases. A chemical reagent widely used for this purpose is bisulfite.

Instead of a chemical conversion, it is furthermore possible to conduct the conversion enzymatically, e.g. by use of methylation-specific cytidine deaminases (German Patent DE 103 31 107 B; PCT/EP2004/007052).

In a preferred embodiment, the quantification or detection of amplified molecules using the described analysis molecule takes place using a post-PCR hybridization technology, such as agarose gel electrophoresis, melting curve analysis, southern blotting, linear array, or micro array.

In another preferred embodiment, the amplification products generated using the described analysis molecule are labeled with a radioactive tag, a mass tag, a chemiluminescence tag or a fluorescence tag. For this, for example but not limited to, amplification is performed by means of one or more labeled nucleotide or one or more labeled primers. This allows for the analysis of the product by for example but not limited to HPLC, capillary electrophoresis, or mass spectroscopy. A person skilled in the art knows further suitable product analysis methods.

It is well known in the art that bisulfite specifically reacts with unmethylated cytosine regardless of the surrounding sequence. Upon subsequent alkaline hydrolysis, the unmethylated cytosine is converted to uracil, while 5-methylcytosine remains unmodified during this treatment (Shapiro et al. (1970) Nature 227: 1047)). Uracil exhibits the same base pairing behavior as thymine; that is, it hybridizes with adenine. 5-methylcytosine does not change its chemical properties under this treatment and therefore still hybridizes with guanine. Consequently, under bisulfite treatment, the original DNA is converted in such a manner that 5-methylcytosine can now be detected as cytosine whereas those cytosines that were unmethylated in the original DNA can now be detected as uracil, showing the base pair behavior of thymine. Due to this, 5-methylcytosine, which originally could not be distinguished from cytosine by its hybridization behavior, can now be differentiated from cytosine using conventional molecular biological techniques, such as amplification, hybridization, or sequencing, all of which are based on base pairing.

An overview of the further known methods of detecting 5-methylcytosine may be gathered from Fraga FM and Esteller M, Biotechniques (2002) 33(3): 632, 634, 636-649.

The chemical reaction occurring will later be described with reference to figure 2. The term "bisulfite" used herein refers to, but is not limited to, a reagent comprising bisulfite, disulfite, hydrogen sulfite or combinations thereof, useful to distinguish between methylated and unmethylated CpG dinucleotide sequences. Methods to perform this treatment are known in the art.

According to a preferred embodiment, a bisulfite treatment is essentially carried out as described in WO05/038051, WO 2006/040187 or in WO 2006/113770. According to this, in one embodiment DNA is reacted with a bisulfite reagent, **characterized in that** said reaction is carried out in the presence of dioxane or dioxane derivatives or in the presence of n-alkylene glycol compounds, particularly their dialkyl ethers, and especially diethylene glycol dimethyl ether (DME). Preferably, the denaturing solvents are used in concentrations between 1 % and 35 % (v/v). In a preferred embodiment, the n-alkylene glycol compounds, in particular DME are preferably used in concentrations between 1-35% (vol/vol), preferably between 5 and 25%, and most preferably 10% DME.

According to a preferred embodiment, the treatment with a bisulfite comprises a radical scavenger. Preferably the radical scavenger is chromane derivatives, e.g., 6-hydroxy-2,5,7,8,-tetramethylchromane 2-carboxylic acid (also known as: Trolox-C™) or derivative thereof. In a preferred embodiment the concentration for 6-hydroxy-2,5,7,8,-tetramethylchromane 2-carboxylic acid is between 35 and 50 mmol/l. Preferably the radical scavenger is gallic acid (3,4,5- trihydroxy benzoic acid) or a derivative thereof. In a preferred embodiment the concentration for gallic acid and gallic acid derivatives is between 50 and 60 mmol/l.

According to a preferred embodiment, the treatment with a bisulfite comprises a radical scavenger, in particular 6-hydroxy-2,5,7,8,-tetramethylchromane 2-carboxylic acid or gallic acid, and an organic solvent, in particular DME.

Further scavengers usable according to the invention are listed in the patent application WO 01/98528.

The bisulfite conversion can be conducted in a wide temperature range from 0 to 95 °C. However, as at higher temperatures the rates of both the conversion and decomposition of the DNA increase, in a preferred embodiment the reaction temperature lies between 0-80°C, preferably between 30-80°C. Particularly preferred is a range between 50-70 °C; most particularly preferred between 57-65°C. The optimal reaction time of the bisulfite treatment depends on the reaction temperature. The reaction time normally amounts to between 1 and 18 hours (see: Grunau et al. 2001, Nucleic Acids Res. 2001, 29(13):E65-5). The reaction time is ordinarily 4-6 hours for a reaction temperature of 60°C.

In a particularly preferred embodiment of the method according to the invention, the bisulfite conversion is conducted at mild reaction temperatures, wherein the reaction temperature is then clearly increased for a short time at least once during the course of the conversion. In this way, the effectiveness of the bisulfite conversion can be surprisingly clearly be increased. The temperature increases of short duration are named "thermospikes". The "standard" reaction temperature outside the thermospikes is denoted as the basic reaction temperature. The basic reaction temperature amounts to between 0 and 80°C, preferably between 30-80°C, more preferably between 50-70 °C, most preferably between 57-65°C.

The reaction temperature during a thermospike is increased to over 85 °C by at least one thermospike. The optimal number of thermospikes is a function of the basic reaction temperature. The higher the optimal number of thermospikes is, the lower is the basic reaction temperature. Preferably, at least one thermospike is necessary in each case. And, on the other hand, in principle, any number of thermospikes is conceivable. Of course, it must be considered that with a large number of temperature increases, the decomposition rate of the DNA also increases, and an optimal conversion is no longer assured. The preferred number of thermospikes is thus between 1 and 10 thermospikes each time, depending on the basic reaction temperature. A number of two to 5 thermospikes is thus particularly preferred. The thermospikes increase the reaction temperature preferably to 85 to 100°C, particularly preferably to 90-100°C, and most preferably to 94°C-100°C.

The duration in time of the thermospikes also depends on the volume of the reaction batch. It must be assured that the temperature is increased uniformly throughout the total reaction solution. For a 20 µl reaction batch when using a thermocycler a duration between 15 seconds and 1.5 minutes, especially a duration between 20 and 50 seconds is preferred. In a particular preferred embodiment the duration is 30 seconds. Operating on a volume of 100 µl the preferred range lies between 30 seconds and 5 minutes, especially between 1 and 3 minutes. Particularly preferred are 1.5-3 minutes. For a volume of 600 µl, a duration of 1 to 6 minutes, is preferred, especially between 2 and 4 minutes. Particularly preferred is a duration of 3 minutes. A person skilled in the art will easily be able to determine suitable durations of thermospikes in relation to a variety of reaction volumes. The above-described use of thermospikes leads to a significantly better conversion rates in the bisulfite conversion reaction, even when the above-described denaturing solvents are not utilized.

According to the invention, the said treatment of DNA with bisulfite is particularly preferred because it has several important advantages in comparison to other known methods or kits of the state of the art. These advantages are: i) higher yield of converted DNA; ii) a nearly complete conversion of unmethylated cytosine while methylated cytosine remain unchanged; and iii) almost no further fragmentation of DNA. These advantages are based in milder reaction conditions because of i) a thermal denaturation of DNA; ii) a comparably lower bisulfite concentration; iii) a slightly more alkaline pH; and iv) the use of a more efficient and more effective radical scavengers.

In a preferred embodiment, the method of the invention is a method, wherein treating DNA with a bisulfite reagent comprises:
mixing of about 10 to about 250 µl of a solution comprising DNA with about 45 to about 750 µl of bisulfite solution, the bisulfite solution having a pH in the range of about 5.45 to about 5.50 comprising about 4.83 to about 4.93 mol/l hydrogensulfite;
adding about 5 to about 500 µl of an organic radical scavenger solution, the organic radical scavenger solution comprising an organic solvent and about 10 to about 750 mmol/l of 6-hydroxy-2,5,7,8-tetramethyl-chroman-2-carboxylic acid; and
applying a temperature protocol for about 2 to about 18 h, wherein the reaction is conducted in a temperature range of about 0 to about 80°C with about 2 to about 5 additional temperature increases, in each case for about 0.5 to about 10 min, to a temperature of about 85 to about 100°C including an initial temperature increase to a temperature of about 85 to about 100°C.

According to a preferred embodiment, the treatment comprising the use of a bisulfite reagent comprises further:
mixing of about 10 to about 250 µl of a solution comprising DNA with about 45 to about 750 µl of bisulfite solution, the bisulfite solution having a pH in the range of about 5.45 to about 5.50 comprising about 4.83 to about 4.93 mol/l hydrogensulfite;
adding about 5 to about 500 µl of an organic radical scavenger solution, the organic radical scavenger solution comprising an organic solvent and about 10 to about 750 mmol/l of 6-hydroxy-2,5,7,8-tetramethyl-chroman-2-carboxylic acid; and
applying a temperature protocol for about 2 to about 18 h, wherein the reaction is conducted in a temperature range of about 0 to about 80°C with about 2 to about 5 additional temperature increases, in each case for about 0.5 to about 10 min, to a temperature of about 85 to about 100°C including an initial temperature increase to a temperature of about 85 to about 100°C.

In a particular preferred embodiment, the method of the invention is a method wherein treating DNA with a bisulfite reagent comprises:
mixing of about 50 to about 150 µl of a solution containing the DNA with about 95 to about 285 µl of the bisulfite solution;
adding about 15 to about 45 µl of DME solution, the DME solution comprising about 500 mmol/l of 6-hydroxy-2,5,7,8-tetramethyl-chroman-2-carboxylic acid dissolved in diethyleneglycoldimethylether; and
applying a temperature protocol for about 3 to about 16 h, wherein the reaction is conducted in a temperature range of about 57 to about 65°C with about 2 to about 5 additional temperature increases, in each case for about 3 to about 5 min, to a temperature of about 94 to about 100°C including an initial temperature increase to a temperature of about 94 to about 100°C.

According to a particular preferred embodiment, the bisulfite treatment of DNA comprises:
mixing of about 50 to about 150 µl of a solution containing the DNA with about 95 to about 285 µl of the bisulfite solution;
adding about 15 to about 45 µl of DME solution, the DME solution comprising about 500 mmol/l of 6-hydroxy-2,5,7,8-tetramethyl-chroman-2-carboxylic acid dissolved in diethyleneglycoldimethylether; and
applying a temperature protocol for about 3 to about 16 h, wherein the reaction is conducted in a temperature range of about 57 to about 65°C with about 2 to about 5 additional temperature increases, in each case for about 3 to about 5 min, to a temperature of about 94 to about 100°C including an initial temperature increase to a temperature of about 94 to about 100°C.

The said treatment with a bisulfite leads to a sulfonation, a deamination, or both of unmethylated cytosine. Deamination is a spontaneous process in an aqueous solution and leads to sulfonated uracil (uracil sulfonate) comprising DNA. No desulfonation occurs yet.

According to a preferred embodiment, uracil sulfonate is desulfonated to uracil by heating.

According to a preferred embodiment, the DNA after bisulfite treatment and if the case may be after purification is directly subjected to amplification. Suitable methods for purification are known to those skilled in the art. Preferably such a method for purification comprises at least one selected of the group consisting of size exclusion, ultrafiltration, Microcon filter device (Millipore Inc.), filter device, ethanol, propanol, silica surface, silica membrane, magnetic particle, polystyrol particle, positively charged surface, and positively charged membrane, charged membrane, charged surface, charged switch membrane, charged switched surface, column of the ZR DNA Clean & Concentrator-5 Kit (Zymo Research Corporation), column of the Wizard Genomic DNA Purification Kit (Promega U.S.), column of the QlAamp DNA Micro Kit (Quiagen, Inc.), a component of the MagNA Pure Compact Nucleic Acid Isolation Kit (I) Large Volume (Roche Diagnostics GmbH), a component of the QlAamp UltraSens Virus Kit (Quiagen, Inc.), a component of the RTP DNA/RNA Virus Supersense Kit (Invitek Gesellschaft für Biotechnik & Biodesign mbH), a component of the chemagic Viral DNA/RNA Kit special (Chemagen Biopolymer-Technologie Aktiengesellschaft), a component of the chemagic DNA Blood Kit special(Chemagen Biopolymer-Technologie Aktiengesellschaft), a component of the High Pure Viral Nucleic Acid Kit (Roche Diagnostics GmbH), a component of the Puregene DNA Isolation Kit (Gentra Systems, Inc.), a component of the MasterPure™ Complete DNA and RNA Purification Kit (Epicentre Technologies), or a component of the NucliSens^{®} Isolation Kit (bioMérieux SA). In particular preferred, is the purification by Microcon™ columns (Millipore Inc.) and the purification by means of SiMAG/FK-Silanol beads (Chemicell GmbH, Germany, Article Number 1101-01 or 1101-05) or beads of the chemagic Viral DNA/RNA Kit special in a previous step (Chemagen Biopolymer-Technologie Aktiengesellschaft; Article Number 1002).

According to a preferred embodiment, uracil sulfonate becomes desulfonated in the initial heating step. If the case may be, the initial heating step lasts for 5 min, 10 min, 15 min, 20 min, 30 min, 45 min or 60 min in order to allow a complete desulfonation of the uracil sulfonate containing DNA. Preferably, the temperature of the initial heating step lies in the range of 80-100°C and most preferably in the range of 90-95°C. The desulfonation by heating treatment is further **characterized in that** it is performed in low alkaline conditions, preferably in the range of pH 7.5-10, most preferably in the range of pH 8.0-9.0.

According to a preferred embodiment, the treated DNA is cleared from contaminating DNA. For this, the treated DNA comprising sulfonated uracil is brought into contact and incubated with an enzyme which specifically degrades non-sulfonated uracil containing nucleic acids. Such an enzyme is for example but not limited to Uracil-DNA-Glycosylase (UNG; WO 2006/040187).

In a particular preferred embodiment for providing a decontaminated template DNA for polymerase based amplification reactions, the sulfonated and/or deaminated template DNA are mixed with an UNG activity and components required for a polymerase mediated amplification reaction or an amplification based detection assay. After degradation of non-sulfonated uracil containing nucleic acids by use of UNG, the UNG activity is terminated and the template DNA is desulfonated by increased temperature. Subsequently the template DNA is ready to be amplified.

Preferably, degradation, termination, desulfonation and amplification occur in a single tube during a polymerase based amplification reaction and/or an amplification based assay. Preferably such an amplification is performed in the presence of dUTP instead of dTTP.

Preferably, sulfonated and partially or completely deaminated DNA after bisulfite treatment is subjected directly to a polymerase based amplification reaction and/or an amplification based assay without any prior desulfonation. The desulfonation occurs during the initial temperature increase of the amplification reaction.

According to a preferred embodiment, uracil sulfonate is desulfonated to uracil by treatment with an alkaline reagent or solution.

In a preferred embodiment, the desulfonation of bisulfite treated DNA comprises the use of at least one selected from the group consisting of size exclusion, ultrafiltration, Microcon filter device (Millipore Inc.), filter device, ethanol, propanol, silica surface, silica membrane, magnetic particle, polystyrol particle, positively charged surface, and positively charged membrane, charged membrane, charged surface, charged switch membrane, charged switched surface, column of the ZR DNA Clean & Concentrator-5 Kit (Zymo Research Corporation), column of the Wizard Genomic DNA Purification Kit (Promega U.S.), column of the QlAamp DNA Micro Kit (Quiagen, Inc.), a component of the MagNA Pure Compact Nucleic Acid Isolation Kit (I) Large Volume (Roche Diagnostics GmbH), a component of the QlAamp UltraSens Virus Kit (Quiagen, Inc.), a component of the RTP DNA/RNA Virus Supersense Kit (Invitek Gesellschaft für Biotechnik & Biodesign mbH), a component of the chemagic Viral DNA/RNA Kit special (Chemagen Biopolymer-Technologie Aktiengesellschaft), a component of the chemagic DNA Blood Kit special(Chemagen Biopolymer-Technologie Aktiengesellschaft), a component of the High Pure Viral Nucleic Acid Kit (Roche Diagnostics GmbH), a component of the Puregene DNA Isolation Kit (Gentra Systems, Inc.), a component of the MasterPure™ Complete DNA and RNA Purification Kit (Epicentre Technologies), or a component of the NucIiSens^{®} Isolation Kit (bioMérieux SA). In particular preferred embodiment, desulfonation is performed by means of Microcon™ columns (Millipore Inc.). The said columns are particularly preferred because they show the highest yield of recovery of bisulfite treated DNA when applied to desulfonation. In addition they allow a recovery of small bisulfite treated fragments as well as large bisulfite treated fragments as present in a sample after bisulfite treatment. A person skilled in the art knows to select other suitable devices or kits in considering the above specifications and named kits.

Particularly preferred is a desulfonation of bisulfite treated DNA comprising:
adding of about 50 to about 1000 µl of water to the sample after the bisulfite reaction;
applying the mixture onto a Microcon filter device subsequently centrifuging at about 10,000 to about 18,000 x g for about 10 to about 30 min;
washing with about 100 to about 800 µl of about 0.2 mol/l sodium hydroxide, and subsequent centrifuging at about 10,000 to about 18,000 x g for about 6 to about 25 min;
applying of about 100 to about 800 µl of about 0.1 mol/l sodium hydroxide, and subsequent centrifuging at about 10,000 to about 18,000 x g for about 6 to about 25 min;
applying, in 1 to about 8 repetitions, the following: applying of about 100 to about 400 µl water or TE buffer and subsequent centrifuging at about 10,000 to about 18,000 x g for about 6 to about 25 min; and
eluting by application of about 25 to about 200 µl TE buffer preheated to about 15 to about 65°C, incubation for about 1 to about 30 min at a temperature of about 15 to about 65°C, and subsequent inversion of the Microcon filter device and centrifugation at about 500 to about 5,000 x g for about 0.5 to about 30 min.

Particularly preferred is a desulfonation of bisulfite treated DNA comprising:
a) adding of 200 µl water to the sample after the bisulfite reaction,
b) applying the mixture onto a Microcon filter device subsequently centrifuging at about 14,000 x g for about 20 min,
c) washing with about 400 µl of about 0.2 mol/l sodium hydroxide, and subsequent centrifuging at about 14,000 x g for about 10 to about 14 min,
d) applying of about 400 µl of about 0.1 mol/l sodium hydroxide, and subsequent centrifuging at about 14,000 x g for about 10 to about 14 min,
e) applying, in 1 to about 4 repetitions, the following: applying of about 400 µl water or TE buffer and subsequent centrifuging at about 14,000 x g for about 12 min; and
f) eluting by application of about 45 to about 70 µl TE buffer preheated to about 50°C, incubation for about 10 min at a temperature of about 50°C, and subsequent inversion of the Microcon filter device and centrifugation at about 1,000 x g for about 7 min.

Particularly preferred is a desulfonation of bisulfite treated DNA that comprises at least one of the following:
in step b, applying the mixture in portions onto the Microcon filter device in step b;
subsequent to step b, applying of about 400 µl TE buffer, the TE buffer pH 8 containing about 10 mmol/l tris-hydroxymethyl-amino-methan and about 0.1 mmol/l EDTA, subsequent centrifuging at about 14,000 x g for about 12 min;
in step c, incubating the about 0.2 mol/l sodium hydroxide for about 10 min at room temperature;
in step d, incubating the about 0.1 mol/l sodium hydroxide for about 10 min at room temperature.
According to a preferred embodiment, nucleic acid treated chemically or enzymatically is subjected to preamplification according to conventional methods. Suitable methods are known to those skilled in the art, such as but not limited to PCR, primer extension, whole genomic amplification after said treatment, amplification of a subset of genomic sequences after said treatment, or single site specific amplification after said treatment. After said preamplification, an amplification by means of an inventive analysis molecule takes place.

To selectively block the amplification of either template DNA derived from methylated DNA or of template DNA derived from unmethylated DNA, an appropriate analysis molecule needs to be used as described above. Accordingly, for blocking template DNA derived from unmethylated DNA the second region of the analysis molecule contains either an adenine (C rich blocker acting part) or a thymine residue (G rich blocker acting part) at the one or more positions that corresponds to unmethylated cytosine in the nucleic acid to be analyzed. Accordingly, for blocking template DNA derived from methylated DNA, the second region of the analysis molecule contains either a guanine residue (C rich blocker acting part) or a cytosine residue (G rich blocker acting part) at the one or more positions that corresponds to methylated cytosine in the nucleic acid to be analyzed.

Preferred embodiments of the method of the invention have the following advantages. With the present method it is possible to block sequences of low complexity (with a low G/C content), such as those of nucleic acids that have been bisulfite treated, for analyzing a small number of cytosine positions located close to each other. The sensitivity of conventional methods such as the HeavyMethyl (HM) method is many times limited by a sufficient large number of closely located CpG positions. The higher the number of CpG positions that are comprised by a blocker, the more specific is the suppression of undesired nucleic species amplification and the more sensitive is the amplification and analysis of the desired nucleic species. Thus with the present invention, it is possible to detect a specifically methylated nucleic acid species of the Septin 9 locus that is present at only 0.1 % (so 99.9% is a nucleic acid species not of interest) by means of only four cytosine positions located closely to each other. In comparison to this, the conventional HM method needs five cytosine positions in order to block the amplification of the nucleic acid species not of interest with the same efficiency and to detect the nucleic acid species with the same sensitivity (see example 2).
In addition, with the present method it is possible to block sequences of low complexity (with a low G/C content), such as those of nucleic acids that have been bisulfite treated, wherein only a single cytosine position is used to discriminate between the different nucleic acid species.

Furthermore, the underlying problem of the invention is also solved by a kit for analyzing a nucleotide position in a nucleic acid. Preferably, said kit is a kit for methylation analysis. Such a kit comprises an analysis molecule as described above for distinguishing between methylated and unmethylated cytosine nucleotides. In addition, said kit comprises a chemical reagent or an enzyme for treating a nucleic acid, said reagent or enzyme alters the base pairing behavior of methylated cytosine bases and/or unmethylated cytosine bases of the nucleic acid such that methylated cytosine bases become distinguishable from unmethylated cytosine bases. Preferably, said reagent or enzyme alters the base pairing behavior of unmethylated cytosine bases while the base pairing behavior of methylated cytosine bases remains unchanged. Preferably, unmethylated cytosines have the same base pair behavior as uracil after treatment with said reagent or enzyme. Particularly preferred said chemical reagent is bisulfite. Particularly preferred said enzyme is a cytidine-deaminase. Preferably, said kit comprises also suitable means and reagents for bisulfite treatment as described herein; suitable means and reagents for purification of nucleic acid, in particular of genomic DNA or of bisulfite treated DNA; suitable means or reagents for performing an amplification reaction as described herein, preferably for PCR and in particular for real time PCR; or combinations thereof.

A further subject matter of the invention is a kit for the analysis of SNP or mutations. Besides an analysis molecule as described herein, said kit comprises suitable means or reagents for performing an amplification reaction as described herein, preferably for PCR amplification.

Preferably, the described kits may further comprise at least one of the additional components:
- a denaturing reagent and/or solution, for example: dioxane or diethylene glycol dimethylether (DME) or any substance, which is suitable as described herein or as described in WO 05/038051,
- a radical scavenger, for example 6-hydroxy-2,5,7,8-tetramethylchromane 2-carboxylic acid or other radical scavengers as described herein or as described in WO 01/98528 or WO 05/038051,
- at least one additional primer, which is suitable for the amplification of one or more DNA amplificates, in particular as described herein
- a reaction buffer, which is suitable for a bisulfite treatment and/or a PCR reaction, as it is known to those skilled in the art
- a nucleotide, which can be dATP, dCTP, dTTP, dUTP and dGTP or any derivative of these nucleotides, including bases with altered pairing properties,
- MgCl₂ as a substance or in solution, any other magnesium salt and/or any other salt, which can be used to carry out a DNA polymerase replication,
- a DNA polymerase, for example Taq polymerase or any other polymerase with or without proof-reading activity, said polymerase is preferably a heatstable polymerase.

A further subject matter of the invention is the use of an inventive analysis molecule, of an embodiment of the method of the invention or of an inventive kit for the detection and/or quantification of SNP, mutations, or cytosine methylation. In case of cytosine methylation, the use may comprise the analysis of single or of multiple cytosine positions. Said positions are either methylated or unmethylated. Said positions are either co-methylated or not. Said use is in particular preferred wherein a methylation pattern is quantified.

The present invention (an inventive analysis molecule, an embodiment of the inventive method or an inventive kit) can be used for the diagnosis and/or prognosis of adverse events for patients or individuals, whereby "diagnosis" is meant to refer to diagnosis of an adverse event, a predisposition for an adverse event and/or a progression of an adverse event.

These adverse events belong to at least one of the following categories: undesired drug interactions; cancer diseases; CNS malfunctions; damage or disease; symptoms of aggression or behavioral disturbances; clinical; psychological and social consequences of brain damages; psychotic disturbances and personality disorders; dementia and/or associated syndromes; cardiovascular disease, malfunction or damage; malfunction, damage or disease of the gastrointestinal tract; malfunction, damage or disease of the respiratory system; lesion, inflammation, infection, immunity and/or convalescence; malfunction, damage or disease of the body as an abnormality in the development process; malfunction, damage or disease of the skin, of the muscles, of the connective tissue or of the bones; endocrine and metabolic malfunction, damage or disease; headaches or sexual malfunction.

The present invention (an inventive analysis molecule, an embodiment of the inventive method or an inventive kit) can also be used for distinguishing cell types and/or tissues and/or for investigating cell differentiation as well as, with regard to methylation, for identifying an indication-specific target in a nucleic acid, which is defined by a difference in the methylation status of a nucleic acid derived from a diseased tissue in comparison to the methylation status of a nucleic acid derived from a healthy tissue.

A particular aspect of the invention refers to an analysis molecule for the analysis of a nucleotide position in a nucleic acid, wherein the nucleotide position is in a first state or an alternative second state, comprising
- a first region for priming a nucleic acid amplification reaction by hybridizing with the nucleic acid to be analyzed, and
- a second region for blocking the amplification reaction if the nucleotide position is either in the first state or in the second state.

In an preferred embodiment, the first region of the inventive analysis molecule is located on the 3' side and the second region is located on the 5' side of the inventive analysis molecule.

In an preferred embodiment of an inventive analysis molecule, the first region is separated from the second region by a third region for enabling the second region to sterically move freely with respect to the first region to allow the formation of a loop structure, and/or for stopping a polymerase during an elongation reaction to prevent the synthesis of a strand that is reverse complementary to the second region.

In an preferred embodiment, an analysis molecule is preferred, wherein
- the first region is able to hybridize with a first sequence portion of the nucleic acid to be analyzed for priming an amplification reaction, and
- the second region is able to hybridize with the product of the amplification reaction primed with the first region of the analysis molecule.

In an preferred embodiment, an analysis molecule is preferred, wherein the first region and/or the second region is an oligonucleotide.

In an preferred embodiment, an analysis molecule is preferred, wherein the third region comprises an alkylic group, a PNA or an abasic site.

In an preferred embodiment, an analysis molecule is preferred, wherein the first region and/or the second region comprises a modification for improving the selectivity and/or stability of the binding with the product of the amplification reaction, a LNA-, a PNA-, a gripNA-, a O-MeRNA-nucleotide, a DNA intercalating molecule, a minor groove binder, or combinations thereof.

In an preferred embodiment, an analysis molecule is preferred, wherein the second region comprises a position that corresponds to a single nucleotide polymorphism (SNP) in the nucleic acid to be analyzed. Said analysis molecule is in particular preferred for the analysis of a genotype (allelic variant) of interest through blocking the amplification of the genotype (allelic variant) not of interest.

In an preferred embodiment, an analysis molecule is preferred, wherein the second region comprises a thymine or an adenine residue at a position that corresponds to an unmethylated cytosine in the nucleic acid to be analyzed, for analyzing the methylation state of the nucleotide position through blocking the amplification of the unmethylated nucleic acid.

In an preferred embodiment, an analysis molecule is preferred, wherein the second region comprises a guanine or a cytosine residue at a position that corresponds to a methylated cytosine in the nucleic acid to be analyzed, for analyzing the methylation state of the nucleotide position through blocking the amplification of the methylated nucleic acid.

In particular aspects the invention refers to the use of an inventive analysis molecule for the analysis of a nucleotide position in a nucleic acid, wherein the nucleotide position can be in a first state or an alternative second state.

In particular aspects the invention refers to a method for the analysis of a nucleotide position in a nucleic acid, comprising
- providing an inventive analysis molecule, and
- amplifying the nucleic acid.

According to a preferred embodiment, amplifying the nucleic acid is performed with a second primer.

According to a preferred embodiment, the second primer is an inventive analysis molecule.

According to a preferred embodiment, amplifying the nucleic acid is performed in the presence of a labeled probe for generating a detectable signal upon binding to the amplification product.

According to a preferred embodiment, amplifying the nucleic acid is performed through a real time polymerase chain reaction.

A particular preferred embodiment, comprises the additional step prior to amplifying the nucleic acid by means of an analysis molecule of treating the nucleic acid with a chemical reagent or an enzyme containing solution, whereby the base pairing behavior of methylated cytosine bases and/or unmethylated cytosine bases of the nucleic acid are altered such that methylated cytosine bases become distinguishable from unmethylated cytosine bases.

According to particular preferred embodiment, the chemical reagent is a bisulfite.

According to particular preferred embodiment, the analysis molecule is an analysis molecule for analyzing the methylation state of a nucleotide position. In particular an analysis molecule is preferred, wherein the second region comprises a thymine or an adenine residue at a position that corresponds to an unmethylated cytosine in the nucleic acid to be analyzed, for analyzing the methylation state of the nucleotide position through blocking the amplification of the unmethylated nucleic acid. In particular an analysis molecule is preferred, wherein the second region comprises a guanine or a cytidine residue at a position that corresponds to a methylated cytosine in the nucleic acid to be analyzed, for analyzing the methylation state of the nucleotide position through blocking the amplification of the methylated nucleic acid.

In particular aspects the invention refers to a kit for analyzing the methylation state of a nucleotide position in a nucleic acid, comprising
- an inventive analysis molecule,
- a chemical reagent or an enzyme for treating a nucleic acid, which alters the base pairing behavior of methylated cytosine bases and/or unmethylated cytosine bases of the nucleic acid such that methylated cytosine bases become distinguishable from unmethylated cytosine bases. For this, an analysis molecule is particularly preferred, wherein the second region comprises a thymine or an adenine residue at a position that corresponds to an unmethylated cytosine in the nucleic acid to be analyzed, for analyzing the methylation state of the nucleotide position through blocking the amplification of the unmethylated nucleic acid. For this, an analysis molecule is particularly preferred, wherein the second region comprises a guanine or a cytidine residue at a position that corresponds to a methylated cytosine in the nucleic acid to be analyzed, for analyzing the methylation state of the nucleotide position through blocking the amplification of the methylated nucleic acid.

According to a preferred variant of the inventive kit, the chemical reagent is a bisulfite.

Subject matter of the invention is an analysis molecule for the analysis of a nucleotide position in a nucleic acid, wherein the nucleotide position is in a first state or an alternative second state, comprising
- a first region for priming a nucleic acid amplification reaction by hybridizing with the nucleic acid to be analyzed, and
- a second region for blocking the amplification reaction if the nucleotide position is either in the first state or in the second state.

Preferably, the first region of the analysis molecule is located on the 3' side and the second region is located on the 5' side of the molecule.

Preferably, the first region of the analysis molecule is separated from the second region by a third region for enabling the second region to sterically move freely with respect to the first region to allow the formation of a loop structure, and/or for stopping a polymerase during an elongation reaction to prevent the synthesis of a strand that is reverse complementary to the second region.

Preferably,
- the first region of the analysis molecule can hybridize with a first sequence portion of the nucleic acid to be analyzed for priming an amplification reaction, and
- the second region of the analysis molecule can hybridize with the product of the amplification reaction primed with the first region of the analysis molecule.

Preferably, the first region and/or the second region of the analysis molecule is an oligonucleotide.

Preferably, the third region of the analysis molecule comprises an alcylic group, a PNA or an abasic site.

Preferably, the first region or the second region of the analysis molecule comprises a modification for improving the selectivity and/or stability of the binding with the product of the amplification reaction, such as a LNA-, a PNA-, a gripNA-, a O-MeRNA-nucleotide, or a DNA intercalating molecule, such as a minor groove binder, or combinations thereof.

Preferably, the second region of the analysis molecule comprises a thymidine or an adenosine residue at a position that corresponds to an unmethylated cytosine in the nucleic acid to be analyzed, for analyzing the methylation state of the nucleotide position through blocking the amplification of the unmethylated nucleic acid.

Preferably, the second region of the analysis molecule comprises a guanosine or a cytidine residue at a position that corresponds to a methylated cytosine in the nucleic acid to be analyzed, for analyzing the methylation state of the nucleotide position through blocking the amplification of the methylated nucleic acid.

Subject matter of the invention is further the use of an analysis molecule of the invention for the analysis of a nucleotide position in a nucleic acid, wherein the nucleotide position can be in a first state or an alternative second state.

Subject matter of the invention is further a method for the analysis of a nucleotide position in a nucleic acid, characterized by the steps of
- providing an analysis molecule according to claims 1 to 9, and
- amplifying the nucleic acid.

According to a preferred embodiment, amplifying the nucleic acid is performed with a second primer that is preferably an analysis molecule according to claims 1 to 9.

According to a preferred embodiment, the second primer is an analysis molecule according to claims 1 to 9.

According to a preferred embodiment, amplifying the nucleic acid is performed in the presence of a labeled probe for generating a detectable signal upon binding to the amplification product.

According to a preferred embodiment, amplifying the nucleic acid is performed through a real time polymerase chain reaction.

A preferred embodiment comprises the additional step prior to amplifying the nucleic acid of treating the nucleic acid with a chemical reagent or an enzyme containing solution, whereby the base pairing behavior of methylated cytosine bases and/or unmethylated cytosine bases of the nucleic acid are altered such that methylated cytosine bases become distinguishable from unmethylated cytosine bases.

Preferably, the chemical reagent is a bisulfite.

According to a preferred embodiment, the analysis molecule is an analysis molecule according to the invention, for analyzing the methylation state of a nucleotide position.

Subject matter of the invention is further a kit for analyzing the methylation state of a nucleotide position in a nucleic acid, comprising
- an analysis molecule according to claims 8 or 9,
- a chemical reagent or an enzyme for treating a nucleic acid, which alters the base pairing behavior of methylated cytosine bases and/or unmethylated cytosine bases of the nucleic acid such that methylated cytosine bases become distinguishable from unmethylated cytosine bases.

In a preferred embodiment of the kit, the chemical reagent is a bisulfite.

Subject matter of the invention is further the use of a method of the invention and/or of a kit according to the invention for the analysis of a nucleotide position in a nucleic acid, wherein the nucleotide position can be in a first state or an alternative second state.

### Description of the drawings

### Figure 1:

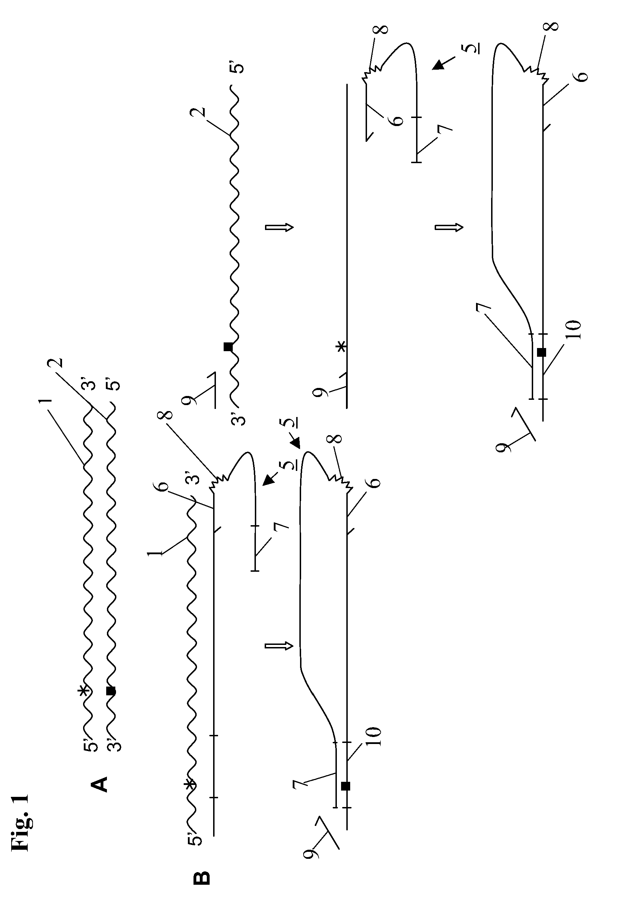

Figure 1 depicts an analysis molecule of the invention for the analysis of a single nucleotide position and one possible application in analyzing a nucleic acid.

Panel A of figure 1 schematically shows a double stranded DNA molecule. The upper first strand 1 is shown in 5' to 3' direction, and the lower second strand 2 being reverse complementary to the upper strand 1 is shown in a 3' to 5' direction (from left to right). In both the first strand 1 and the second strand 2, a single nucleotide position that is to be analyzed is marked by an asterisk in the first strand 1, and by a square in the second strand 2. The asterisk and the square represent nucleotides with bases that are complementary to each other.

Panel B shows the use of an exemplary analysis molecule 5 according to the present invention that is used in an enzymatic amplification reaction in the form of a PCR of the first strand 1 (shown on the left side) and the second strand 2 (shown on the right side) of the DNA shown.

The analysis molecule 5 comprises a first region 6 at its 3' end that can initiate a nucleic acid amplification reaction by hybridizing with first strand 1 that is to be amplified. A second region 7 at the 5' end of the analysis molecule 5 serves as a blocker for blocking the amplification of the strand that is reverse complementary to the first strand 1.

Between the first region 6 and the second region 7 of the analysis molecule 5, a third region 8 is located that serves mainly two functions:
Firstly, it constitutes a barrier for the polymerase through which it cannot penetrate (i.e. the polymerase will dissociate once it encounters the third region 8 of the analysis molecule 5). Secondly, it enables the second region 7 of the molecule 5 to move around virtually freely and enables the hybridization of the second region 7 of the molecule 5 with a nucleic acid generated during the amplification reaction of the first strand 1. This third region 8 preferably consists of a hexaethylenglycol (HEG) spacer.

The first region 1 of the analysis molecule 5 hybridizes at the 3' end of the first strand 1 of the template DNA and thereby initiates a polymerase driven amplification that proceeds along the first strand 1, containing the single nucleotide position that is to be analyzed. At the same time, the second strand 2 is also amplified using a second primer 9 that hybridizes with the second strand 2 at its 3' region. Note that neither the first region 6 of the analysis molecule 5, nor the second primer 9 hybridizes with a sequence that contains the single nucleotide position to be analyzed in the example shown. Therefore, in the first cycle the amplification is performed in an unbiased fashion.

In this example, the generation of a nucleic acid strand that is reverse complementary to the first strand 1 creates a sequence portion 10 that is reverse complementary to the second region 7 of the analysis molecule 5. Therefore, the second region 7 of the analysis molecule 5 hybridizes to the sequence portion 10 forming a double stranded stretch. The sequence portion 10 that the second region 7 of the analysis molecule 5 hybridizes with, overlaps with the binding sequence of the second primer 9. Thereby, the binding of the second primer 9 is prevented and the further amplification is stopped.

The second strand 2 is first amplified by the use of the second primer 9. The first region 6 of the analysis molecule 5 can then hybridize to the 3' end of the molecule generated in this amplification reaction, itself initiating an amplification reaction during which a sequence portion 10 will be generated that the second region 7 of the analysis molecule 5 can bind to.

As will be understood by a person of skill in the art, the second region 7 of the analysis molecule 5 can contain a sequence that perfectly hybridizes with the sequence portion 10, not allowing the second primer 9 to bind and amplify the template DNA. It is, however, also possible to use a sequence in the second region 7 of molecule 5 that differs from the sequence of sequence portion 10, at the single nucleotide position to be analyzed. Therefore, the analysis molecule 5 can be used to distinguish these single nucleotide positions and create an amplification product only in those cases in which the second region 7 of the analysis molecule 5 did not perfectly match with the sequence portion 10.

### Figure 2:

Figure 2 pertains to the method of the invention with regard to the complete conversion of unmethylated cytosine to uracil in a process referred to as bisulfite conversion (bisulfite treatment with subsequent alkaline treatment), which is known in the art. In the first step of this reaction, unmethylated cytosine bases are sulfonated at position C6 at a pH around 5 through a reaction with hydrogensulfite.

The second step is the deamination that takes place rather spontaneously in aqueous solution. Thereby, cytosine sulfonate is converted into uracil sulfonate. The third step is the desulfonation step, which takes place in alkaline conditions, resulting in uracil.

Alternatively, it is also possible to stop the procedure after the sulfonation step and omit the desulfonation step. In this case, the sulfonated nucleic acid may be used as a template for the amplification reaction.

### Figure 3:

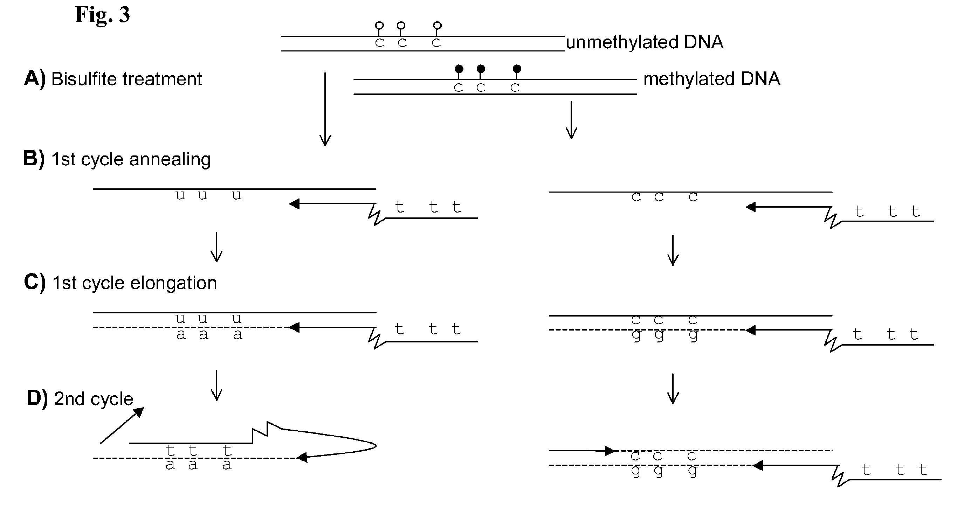

Figure 3 shows the use of an exemplary analysis molecule according to the present invention in the analysis of a nucleotide position. Specifically, several nucleotide positions in the form of cytosine residues are analyzed, which can be in a methylated or an unmethylated state.

Treatment of the genomic DNA with bisulfite leads to the conversion of unmethylated cytosine residues into uracil, which exhibits the same base pairing behavior as thymine. Methylated cytosine residues are not changed by bisulfite and therefore remain as cytosine residues in the genomic DNA. On the left side of panel A, bisulfite converted unmethylated DNA is shown, and on the right side of panel A, bisulfite converted methylated DNA is shown.

The pool of genomic DNA present in the sample to be analyzed mostly contains unmethylated DNA molecules, each containing three unmethylated cytosine residues (left side of panel A). The pool of genomic DNA also contains a small percentage of methylated genomic DNA that is to be analyzed. In this methylated genomic DNA, the three cytosine residues are methylated (right side of panel A).

Accordingly, the analysis molecule according to the invention is used to prevent the amplification of the bisulfite converted nucleic acid that was originally unmethylated, while the bisulfite converted nucleic acid molecule of the same sequence, but with corresponding methylated cytosine residues, is amplified in an enzymatic amplification reaction.

As shown in panel C, the exemplary analysis molecule according to the present invention contains a first region that hybridizes to the bisulfite treated genomic DNA on the 3' side of the nucleotide positions to be analyzed. With this, it initiates a nucleic acid amplification reaction. A second region for blocking the amplification, that binds if a certain methylation status is present in the template genomic DNA, is present at the 5' side of the analysis molecule. Here, the blocker contains thymine residues at the positions in question to block the amplification of the template that stems from the unmethylated DNA. These thymine residues hybridize with adenine residues in the nucleic acid strand that is generated in a first amplification (elongation) round and therefore block amplification of unmethylated genomic DNA. The analysis molecule also contains a third region that allows the second region to develop a fold back confirmation and hybridize intramolecularly with the amplification product.

As shown in panel D, elongation of the genomic DNA template leads to the generation of a DNA strand containing adenine residues in the case of an unmethylated template (left side) and to the incorporation of guanine residues in the DNA strand that is generated from the genomic template (right side).

As can been seen in panel D of figure 3, the hybridization of the second portion of the analysis molecule to a perfectly reversed complementary sequence of the blocker prevents a second (reverse) primer from binding to the generated template strand. Therefore, no amplification product will be generated from the unmethylated genomic DNA in this example. In contrast, the incorporation of guanine residues into the nucleic acid strand generated in the first elongation cycle prevents the second region of the molecule to form a fold back structure and block the binding of a second primer. Therefore, the analysis molecule does not prevent the amplification of the methylated genomic DNA.

In case the amplification is performed as a real time PCR, the generation of an amplification product of the methylated DNA can be followed by an appropriate labeled probe, which is known in the art.

In effect, the analysis molecule according to the invention can be used to analyze the methylation status of a nucleotide position.

### Figure 4:

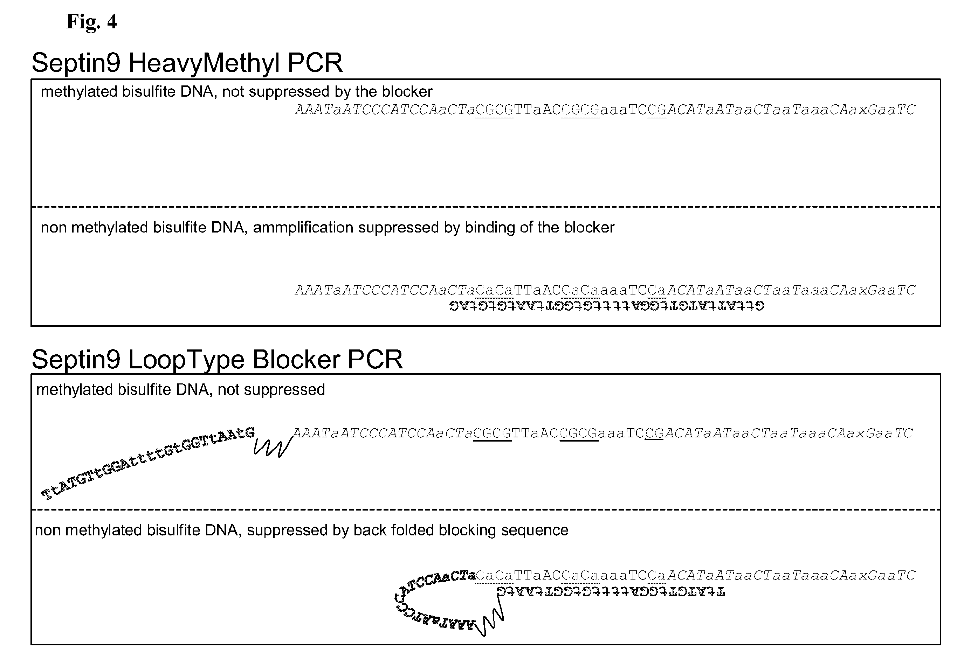

Figure 4 shows the design of an exemplary analysis molecule according to the invention, which is here referred to as "LoopTypeBlocker" (panel B). The third sequence portion is represented by a zick-zacked line. In panel A, the design of a conventional blocker molecule is shown, that can be used for example in a method according to the HeavyMethyl (HM) method, which is described in WO 02/072880 A2. Such a conventional blocker is not linked to a primer. (blocking sequences are displayed in bold; CpG positions are displayed underlined; primer binding regions are displayed italicized)

### Figure 5:

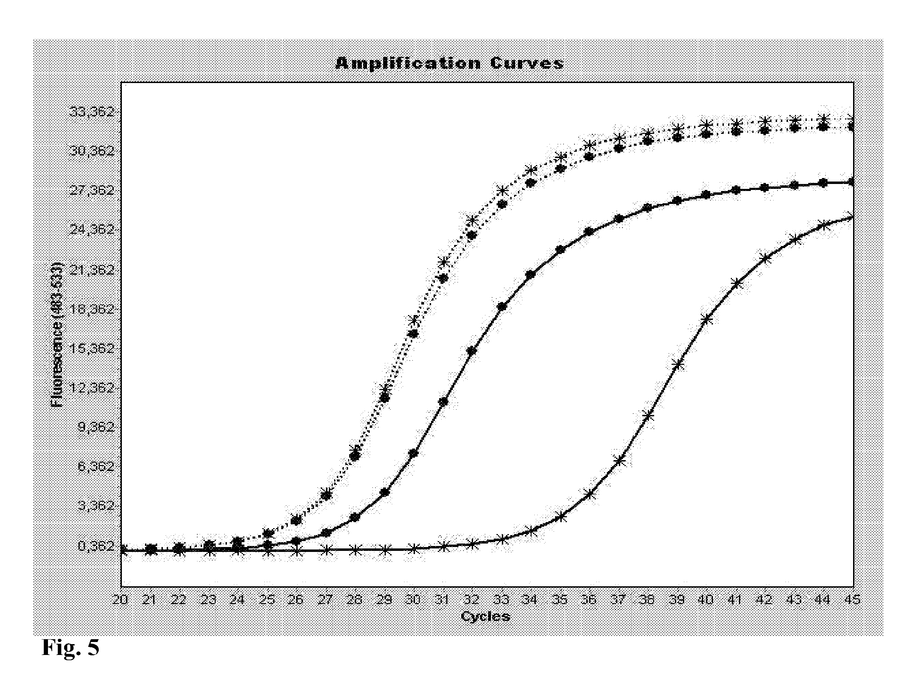

Figure 5 shows real time amplification curves obtained from bisulfite converted DNA using unmodified primers or an unmodified primer and a LoopTypeBlocker. The detection was carried out with SYBR Green I as fluorescence dye. 10 ng methylated bisulfite converted DNA (Circles) and 10 ng unmethylated DNA (Stars) were unbiased amplified with the unmodified primers (dotted lines) showing the same threshold cycle. The PCR reaction mixture containing one unmodified primer and one LoopTypeBlocker (solid lines) results in a favored amplification of methylated DNA. The CT difference of methylated and unmethylated DNA is higher than 7 cycles.

### Figure 6:

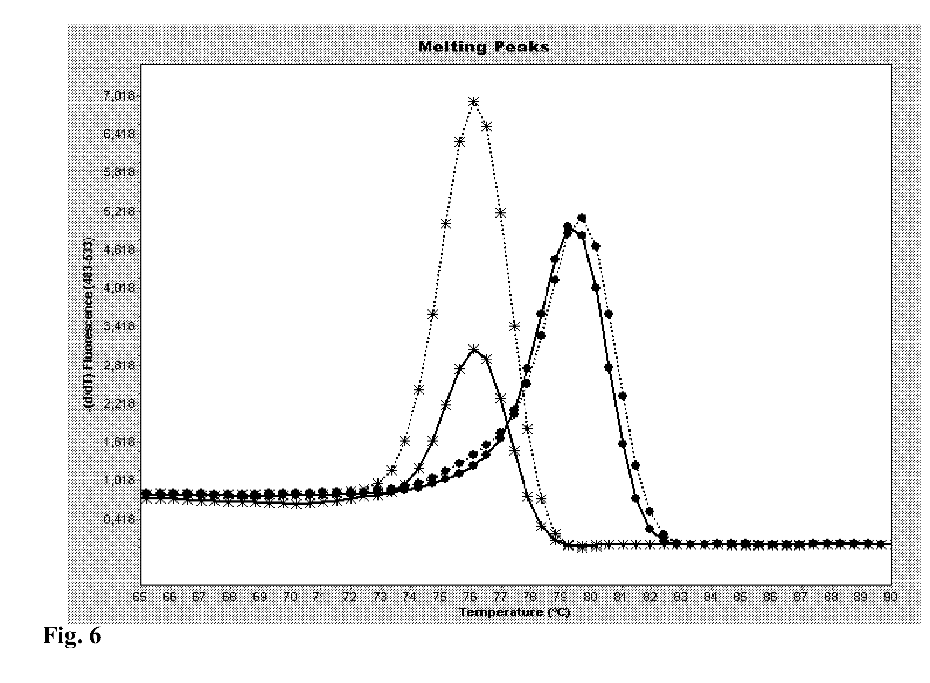

Figure 6 shows a melting curve analysis of the PCR products obtained from bisulfite converted DNA using unmodified primers or an unmodified primer and a LoopTypeBlocker. The detection was carried out with SYBR Green I as fluorescence dye. Due to the different base composition methylated bisulfite converted DNA (circles) typically showed a higher Tₘ than unmethylated DNA (crosses). The melting points obtained from PCR products generated with unmodified primers (dotted lines) are identical to those obtained from products generated with an unmodified primer and a LoopTypeBlocker (solid lines), confirming the sequence identity. The specific suppression of unmethylated DNA results in a lower peak height wherein the LoopTypeBlocker is used.

### Figure 7:

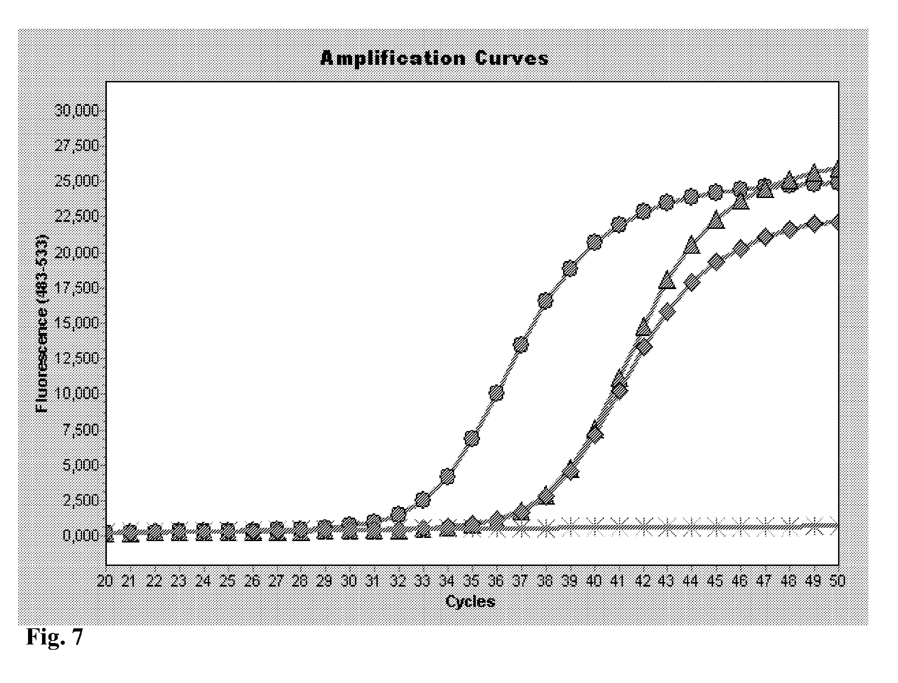

Figure 7 shows a Septin 9 HeavyMethyl real time PCR (Mastermix A) using conventional blocker oligonucleotide. Representative amplification curves are shown for 1 ng (circle) and for 50 pg (triangle) methylated bisulfite converted DNA. No amplification curve was obtained from 50 ng unmethylated bisulfite converted DNA (crosses). The relative sensitivity of 1:1000 was evaluated by a mixture of 50 pg methylated and 50 ng unmethylated DNA (diamonds). The specific detection of methylated sequences is provided by the use of a methylation specific fluorogenic hydrolysis probe.

### Figure 8:

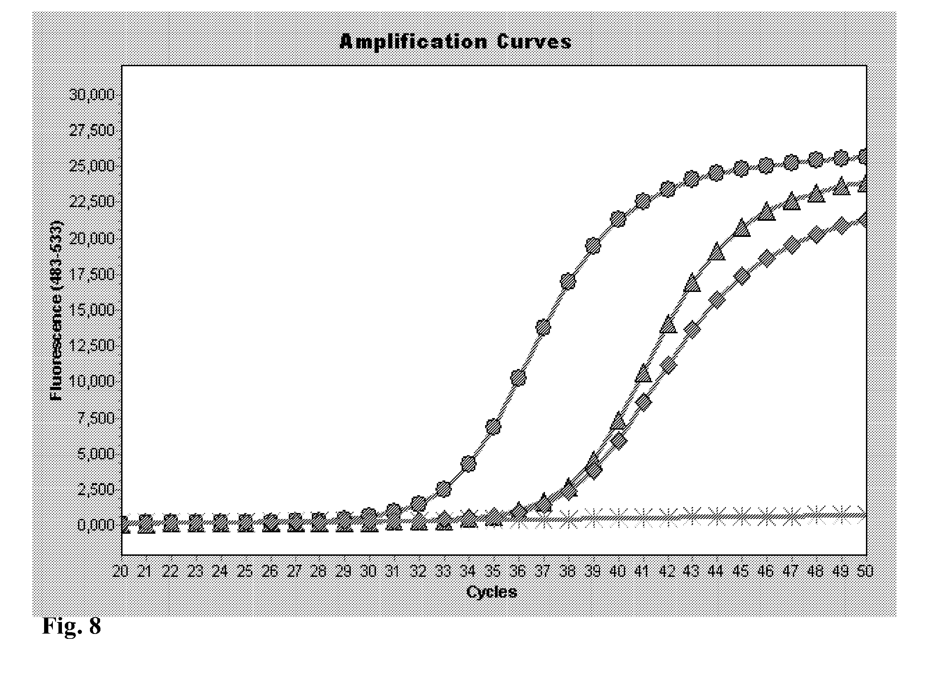

Figure 8 shows real time PCR (Mastermix B) with a LoopTypeBlocker adapted from the described Septin 9 HeavyMethyl PCR. The reaction contained a blocking sequence linked to the forward primer instead of the free blocker oligonucleotide. Representative amplification curves are shown for 1 ng (circle) and for 50 pg (triangle) methylated bisulfite converted DNA. No amplification curve was obtained from 50 ng unmethylated bisulfite converted DNA (crosses). The relative sensitivity of 1:1000 was evaluated by a mixture of 50 pg methylated and 50 ng unmethylated DNA (diamonds). The specific detection of methylated sequences is provided by the use of a methylation specific fluorogenic hydrolysis probe. Please note that the blocking sequence of the LoopTypeBlocker comprises only four CpG positions while the sequence of the blocker of the HeavyMethyl real time PCR of Fig 7 five CpG positions comprises.

### Figure 9:

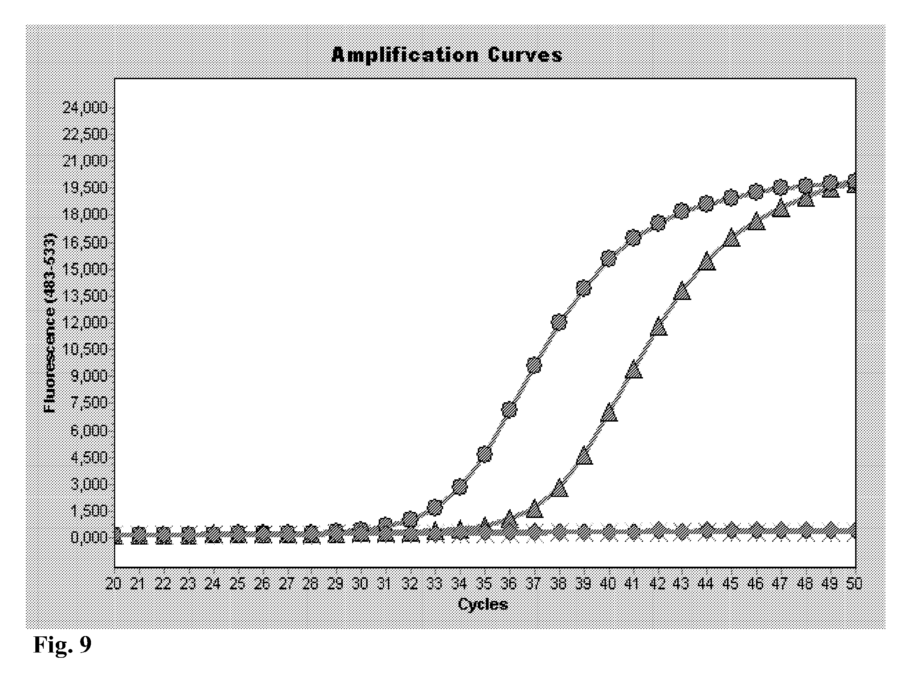

Figure 9 shows a Septin 9 PCR without any blocking element (Mastermix C). The PCR is based on the same primers as described for the HeavyMethyl PCR. However, the PCR mix did not contain the blocker oligonucleotide. Amplification curves are shown for 1 ng (circle) and for 50 pg (triangle) methylated bisulfite converted DNA. No amplification curve were obtained from 50 ng unmethylated bisulfite converted DNA (crosses), because of the use of a fluorogenic hydrolysis probe which is specific for the presence of methylation. Although the mixture of 50 pg methylated and 50 ng unmethylated DNA (diamonds) contained 0,1 % methylated DNA, no methylation specific signal was obtained, demonstrating the limitations of PCR without blocking element. Without the specific inhibition of unmethylated DNA, as described for the LoopTypeBlocker PCR, the methylated template cannot be detected from a 1:1000 mixture with a methylation specific fluorogenic hydrolysis probe.

### DEFINITIONS

In particular aspects, the term "co-methylated" refers to, but is not limited to, the methylation of a group of CpG positions, wherein all of said positions are present either in the methylated state or in the unmethylated state in a single nucleic acid molecule. Preferably, said group consists of at least 2, 3 ,4, 5, 6, 7, 8, 9, 10, 15 or 20) CpG positions.

In particular aspects, the term "methylation pattern" refers to, but is not limited to, the presence or absence of methylation of one or more nucleotides. Thereby said one or more nucleotides are comprised in a single nucleic acid molecule. Said one or more nucleotides have the ability of being methylated or being non-methylated. The term "methylation status" may also be used, wherein only a single nucleotide is considered. A methylation pattern can be quantified, wherein it is considered over more than one nucleic acid molecule.

The term "Loop-Type-Blocker" as used herein refers to an inventive analysis molecule.

The term "bisulfite DNA" as used herein refers to nucleic acid treated with bisulfite according to known methods. Particularly preferred is nucleic acid treated with bisulfite as described herein.

### EXAMPLES

### Example 1: SYBRgreen based methylation specific Detection of Septin 9 using a methylation specific Real Time PCR

### Introduction

In this example, bisulfite converted DNA of the Septin 9 gene was amplified using an unbiased primer pair for the Septin 9 promotor region and compared to the biased amplification using an analysis molecule according to the invention (a "LoopTypeBlocker"). The double-strand DNA specific dye SYBR Green was used to monitor the generation of PCR products in a real time PCR. The use of the LoopTypeBlocker results in a strong suppression of the amplification of unmethylated bisulfite converted DNA, whereas the methylated DNA is preferably amplified.

### Materials and Methods

1 µg human genomic DNA from peripheral blood lymphocytes (PBL DNA, Roche Diagnostics, Penzberg Germany) and 1 µg universal methylated human DNA (GpGenome^{™} Universal Methylated DNA, Chemicon International, Temecula USA) were applied to a bisulfite conversion reaction using the EpiTect Bisulfite Kit (Qiagen, Hilden Germany). 2 µl of the purified DNA were measured twice in the NanoDrop instrument to obtain the mean absorption at 260 nm. The DNA concentration was calculated based on the average extinction of double stranded DNA to be 1.0(A260) = 50 µg/ml. The bisulfite conversion was confirmed through a bisulfite DNA specific reference PCR as previously described (EP05075404). 10 ng of each DNA were used as template DNA in two different Real Time PCRs. The reactions were performed in a total volume of 25 µl containing the following components:
- 10 µl of template DNA
- 12.5 µl Quantitect Multiplex NoROX PCR Kit (Qiagen, Hilden Germany)
- 0.3 µmol/l forward primer (SEQ ID NO 1, Table 1)
- 0.3 µmol/l reverse primer (SEQ ID NO 2, Table 1)
- SYBR Green I dye in a final dilution of 1:40000 (Invitrogen, Carlsbad USA)

The samples were also analyzed using the LoopTypeBlocker combined with the same reverse primer:
- 10 µl of template DNA
- 12.5 µl Quantitect Multiplex NoROX (Qiagen, Hilden Germany)
- 0.3 µmol/l LoopTypeBlocker (SEQ ID NO 5, Table 1)
- 0.3 µmol/l reverse primer (SEQ ID NO 2, Table 1)

The PCR were performed in the LightCycler LC480 (Roche Diagnostics, Penzberg Germany) according to the following temperature time profile:
- Activation: 30 min at 95 °C
- 50 cycles: 10 sec at 95 °C
   30 sec at 56 °C
   10 sec at 72 °C

Subsequently, a melting curve analysis was performed using the following profile: 10 sec at 95 °C, 10 sec at 40 °C, followed by a melting from 40 - 95 °C with a heating rate of 0.1 °C/sec and continuous detection at 533 nm.

The primers of the PCRs (SEQ ID NO 1 or SEQ ID NO 5, and SEQ ID NO 2, Table 1) amplify a fragment of 65 bp of the Septin 9 gene (SEQ ID NO 6, nucleotide 9295830 to nucleotide 9295894 of GenBank Accession Number NT_010641.15). The detection was carried out during the extension step at 72 °C at the 533 nm channel (filter setup 483-533). The threshold cycle (CT) was calculated according to the "second derivative maximum" method by means of the LightCycler 480 software.

### Results

The obtained amplification curves of both PCRs are shown in figure 5. With unmodified primer, both methylated and unmethylated DNA was amplified in a same manner and resulted in almost the same threshold cycle of 26.3 and 26.2, respectively (Table 1). The LoopTypeBlocker (SEQ ID NO 5) combined with the forward primer resulted in an amplification bias for methylated DNA of a factor higher than 100. This can be calculated from the CT difference of 7.3 between methylated and unmethylated DNA, assuming that 1 CT difference is equal to 2fold difference of the DNA amount (Table 2). The melting curve analysis confirmed the identity of the amplified DNA. The same Tₘ was obtained for methylated and unmethylated DNA using the unbiased PCR and the LoopTypeBlocker PCR (Figure 6).

### Example 2: Sensitive Detection of a methylated Septin 9 sequence using a LoopTypeBlocker based real time PCR

### Introduction

In order to reach the goal of a DNA methylation analysis for clinical application, sensitive detection of tumor derived methylated DNA in bodily fluids, such as serum, plasma or urine, needs to be achieved. Because the majority of the extracted DNA originates from healthy cells, the challenge is the sensitive detection of methylated DNA in a high background of unmethylated DNA. In this example, it was demonstrated that the use of a LoopTypeBlocker allows the detection of methylated DNA in a background of unmethylated DNA that is present in at least 1000 fold excess. The experimental data show an equivalent performance of a Septin 9 LoopTypeBlocker PCR compared to a conventional HeavyMethyl PCR for Septin 9.

### Materials and Methods

Bisulfite conversion and quantification of human genomic DNA from peripheral blood lymphocytes (PBL DNA, Roche Diagnostics, Penzberg Germany) and universal methylated human DNA (GpGenome^{™} Universal Methylated DNA, Chemicon International, Temecula USA) was performed as described in example 1. As template, different DNA solutions were prepared containing 1 ng or 50 pg methylated DNA or 50 ng unmethylated DNA (PBL) in 10 µl each. Each of the samples was measured in two replicates.
The HeavyMethyl PCR was performed in a total volume of 25 µl containing following components (Mastermix A):
- 10 µl of template DNA
- 12.5 µl Quantitect Multiplex NoROX (Qiagen, Hilden Germany)
- 0.3 µmol/l forward primer PCR2 (SEQ ID NO 1)
- 0.3 µmol/l reverse primer PCR2 (SEQ ID NO 2)
- 4 µmol/l blocker oligonucleotide (SEQ ID NO 3)
- 0.1 µmol/l Taqman® probe (SEQ ID NO 4)

The LoopTypeBlocker PCR was performed in a total volume of 25 µl containing following components (Mastermix B):
- 10 µl of template DNA
- 12.5 µl Quantitect Multiplex NoROX PCR Kit (Qiagen, Hilden Germany)
- 0.3 µmol/l LoopTypeBlocker (SEQ ID NO 5)
- 0.3 µmol/l reverse primer (SEQ ID NO 2)
- 0.1 µmol/l Taqman® probe (SEQ ID NO 4)

A real time PCR without any blocker oligonucleotide was performed in a total volume of 25 µl containing following components (Mastermix C):
- 10 µl of template DNA
- 12.5 µl Quantitect Multiplex NoROX (Qiagen, Hilden Germany)
- 0.3 µmol/l forward primer PCR2 (SEQ ID NO 1)
- 0.3 µmol/l reverse primer PCR2 (SEQ ID NO 2)
- 0.1 µmol/l Taqman® probe (SEQ ID NO 4)

All PCRs were performed in the LightCycler LC480 (Roche Diagnostics Penzberg Germany) according to the following temperature time profile:
- Activation: 30 min at 95 °C
- 50 cycles: 10 sec at 95 °C
   30 sec at 56 °C
   10 sec at 72 °C

The detection was carried out during the annealing step at 56 °C at the 533 nm channel (filter setup 483-533). The threshold cycles (CT) were calculated according to the "second derivative maximum" method by means of the LightCycler 480 software.

### Results

Under all three conditions (Mastermix A, B and C) 1 ng and 50 pg universally methylated bisulfite treated DNA was reproducibly amplified and detected, but no signals were obtained from 50 ng unmethylated DNA (Table 3). However, 50 pg methylated DNA mixed in a 1000fold excess of unmethylated DNA was detected only if the PCR contained a blocking element. Using both, conventional HeavyMethyl PCR as well as the LoopTypeBlocker PCR, 50 pg methylated DNA in a mixture with 50 ng PBL DNA were detected with almost the same CT value. Also, the signal intensity of the real time PCR obtained from the 1:1000 mixture was comparable between HeavyMethyl and LoopTypeBlocker PCR. In contrast to that, no amplification signal was obtained in the real time PCR without any blocking element (Mastermix C). The blocker oligonucleotide of the HeavyMethyl PCR spans over 5 CpG positions, whereas the LoopTypeBlocker covers only 4 out of the 5 CpGs. However, the results showed the same relative sensitivity and PCR performance and therefore demonstrate the potential of the LoopTypeBlocker technology. Representative amplification curves of all three real time PCRs are shown in Figures 7, 8, and 9 for the mastermixes A, B and C. The mean CTs out of two measurements are summarized in Table 3.

**Table 1: Sequences of oligonucleotides described in the examples (all supplied by Biomers.net GmbH, Germany)**

| **SEQ-ID NO** | **Name** | **Sequence** |
|---|---|---|
| 1 | Sep9.102 | 5'-AAATaATCCCATCCAaCTa |
| 2 | Sep9.100 | 5'-GAtt-X-GtTGtttAttAGttATtATGT |
| 3 | Sep9.8B1 | 5'-GttATtATGTtGGAttttGtGGTtAAtGtGtAG-C3 |
| 4 | Sep9.10taq4 | 5'-FAM-TTaACCGCGaaaTCCGAC-BHQ1 |
| 5 | Sep9.LTB | 5'–TtATGTtGGAttttGtGGTtAAtG-HEG- AAATaATCCCATCCAaCTa |
| 6 | Sep9 amplicon | 5’GAAGTtXGAAATGATtttATttAGtTGCGCGTTGAtCGCGGGG TtCGAtATGATGGtTGGTGG |

| | | |
|---|---|---|
| C3 = 3'OH-Propyl-Modification, FAM = Carboxyfluorescein label, BHQ1 = BlackHoleQuencher 1, X = abasic site by Tetrahydrofuran modification (also known as dSpacer), HEG = Hexaethylenglycol. Small written t's represent cytosines converted by bisulfite treatment, and small a's represent adenine s in the reverse complementary strand, resulting from amplification of converted cytosines. | | |

**Table 2 CTs obtained from 10 ng bisulfite converted DNA in a SYBR Green based real time PCR using unmodified primers and the LoopTypeBlocker PCR.**

| **Template DNA** | **PCR with unmodified primers** | **LoopTypeBlocker PCR** |
|---|---|---|
| 10 ng methylated bisulfite treated DNA | 26.3 | 27.9 |
| 10 ng methylated bisulfite treated DNA | 26.2 | 35.2 |
| No template control | - | - |

**Table 3 Mean CTs out of two replicates obtained with three different real time PCRs for Septin 9.**

| **Template DNA** | **HeavyMethyl PCR (Mastermix A)** | **LoopTypeBlocker PCR (Mastermix B)** | **PCR without blocker (Mastermix C)** |
|---|---|---|---|
| 1 ng methylated DNA | 33.1 | 33.2 | 33.2 |
| 50 pg methylated DNA | 37.9 | 37.8 | 37.3 |
| 50 ng PBL DNA | no signal | no signal | no signal |
| Mix of 50 pg methylated and 50 ng PBL DNA (1:1000) | 37.7 | 37.9 | no signal |

## Claims

1. An analysis molecule for the analysis of a nucleotide position in a nucleic acid, wherein the nucleotide position is in a first state or an alternative second state, comprising
- a first region for priming a nucleic acid amplification reaction by hybridizing with the nucleic acid to be analyzed, and
- a second region for blocking the amplification reaction if the nucleotide position is either in the first state or in the second state.

2. The analysis molecule according to claim 1, wherein the first region of the molecule is located on the 3' side and the second region is located on the 5' side of the molecule.

3. The analysis molecule according to claim 1 or 2, wherein the first region is separated from the second region by a third region for enabling the second region to sterically move freely with respect to the first region to allow the formation of a loop structure, and/or for stopping a polymerase during an elongation reaction to prevent the synthesis of a strand that is reverse complementary to the second region.

4. The analysis molecule according to claim 1-3, wherein
- the first region is able to hybridize with a first sequence portion of the nucleic acid to be analyzed for priming an amplification reaction, and
- the second region is able to hybridize with the product of the amplification reaction primed with the first region of the analysis molecule.

5. The analysis molecule according to claim 1-4, wherein the first region and/or the second region comprises a modification for improving the selectivity and/or stability of the binding with the product of the amplification reaction, a LNA-, a PNA-, a gripNA-, a O-MeRNA-nucleotide, a DNA intercalating molecule, a minor groove binder, or combinations thereof.

6. The analysis molecule according to claim 1-5, wherein the second region comprises a position that corresponds to a single nucleotide polymorphism (SNP) in the nucleic acid to be analyzed.

7. The analysis molecule according to claim 1-6, wherein the second region comprises a thymine or an adenine residue at a position that corresponds to an unmethylated cytosine in the nucleic acid to be analyzed, for analyzing the methylation state of the nucleotide position through blocking the amplification of the unmethylated nucleic acid; or wherein the second region comprises a guanine or a cytosine residue at a position that corresponds to a methylated cytosine in the nucleic acid to be analyzed, for analyzing the methylation state of the nucleotide position through blocking the amplification of the methylated nucleic acid.

8. A method for the analysis of a nucleotide position in a nucleic acid, comprising
- providing an analysis molecule according to claim 1-7, and
- amplifying the nucleic acid.

9. The method according to claim 8, wherein amplifying the nucleic acid is performed with a second primer that is preferably an analysis molecule according to claim 1-7.

10. The method according to claim 8 or 9, wherein amplifying the nucleic acid is performed through a real time polymerase chain reaction.

11. The method according to claim 8-10, with the additional step prior to amplifying the nucleic acid by means of an analysis molecule of treating the nucleic acid with a chemical reagent or an enzyme containing solution, whereby the base pairing behavior of methylated cytosine bases and/or unmethylated cytosine bases of the nucleic acid are altered such that methylated cytosine bases become distinguishable from unmethylated cytosine bases.

12. A kit for analyzing the methylation state of a nucleotide position in a nucleic acid, comprising
- an analysis molecule according to claim 1-7; and
- a chemical reagent or an enzyme for treating a nucleic acid, which alters the base pairing behavior of methylated cytosine bases and/or unmethylated cytosine bases of the nucleic acid such that methylated cytosine bases become distinguishable from unmethylated cytosine bases.

13. Use of an analysis molecule of claim 1-7, a method of claim 8-11 and/or of a kit of claim 12 for the analysis of a nucleotide position in a nucleic acid, wherein the nucleotide position can be in a first state or an alternative second state.


SEQUENCE LISTING

<110>  Epigenomics AG

<120>  An analysis molecule and method for the analysis of a nucleotide
       position in a nucleic acid

<130>  P309EP

<140>  07022417.5
<141>  2007-11-19

<160>  12

<170>  PatentIn version 3.3

<210>  1
<211>  19
<212>  DNA
<213>  Artificial

<220>
<223>  Oligonucleotide

<400>  1
aaataatccc atccaacta                                                    19


<210>  2
<211>  26
<212>  DNA
<213>  Artificial

<220>
<223>  Oligonucleotide


<220>
<221>  misc_feature
<222>  (5)..(5)
<223>  abasic site by Tetrahydrofuran modification (also known as
       dSpacer)

<400>  2
gattngttgt ttattagtta ttatgt                                            26


<210>  3
<211>  33
<212>  DNA
<213>  Artificial

<220>
<223>  Oligonucleotide


<220>
<221>  misc_feature
<222>  (33)..(33)
<223>  C3: 3'OH-Propyl-Modification

<400>  3
gttattatgt tggattttgt ggttaatgtg tag                                    33


<210>  4
<211>  18
<212>  DNA
<213>  Artificial

<220>
<223>  Oligonucleotide


<220>
<221>  misc_feature
<222>  (1)..(1)
<223>  FAM: Carboxyfluorescein label

<220>
<221>  misc_feature
<222>  (18)..(18)
<223>  BHQ1: BlackHoleQuencher 1

<400>  4
ttaaccgcga aatccgac                                                    18


<210>  5
<211>  43
<212>  DNA
<213>  Artificial

<220>
<223>  Oligonucleotide


<220>
<221>  misc_feature
<222>  (24)..(24)
<223>  3' HEG: Hexaethylenglycol linker

<400>  5
ttatgttgga ttttgtggtt aatgaaataa tcccatccaa cta                        43


<210>  6
<211>  63
<212>  DNA
<213>  Artificial

<220>
<223>  Oligonucleotide


<220>
<221>  misc_feature
<222>  (7)..(7)
<223>  abasic site by Tetrahydrofuran modification (also known as
       dSpacer)

<400>  6
gaagttngaa atgattttat ttagttgcgc gttgatcgcg gggttcgata tgatggttgg      60

tgg                                                                    63


<210>  7
<211>  65
<212>  DNA
<213>  Artificial

<220>
<223>  Oligonucleotide


<220>
<221>  misc_feature

<222>  (60)..(60)
<223>  abasic site by Tetrahydrofuran modification (also known as
       dSpacer)

<400>  7
aaataatccc atccaactac gcgttaaccg cgaaatccga cataataact aataaacaan      60

gaatc                                                                  65


<210>  8
<211>  33
<212>  DNA
<213>  Artificial

<220>
<223>  Oligonucleotide

<400>  8
gttattatgt tggattttgt ggttaatgtg tag                                   33


<210>  9
<211>  65
<212>  DNA
<213>  Artificial

<220>
<223>  Oligonucleotide


<220>
<221>  misc_feature
<222>  (60)..(60)
<223>  abasic site by Tetrahydrofuran modification (also known as
       dSpacer)

<400>  9
aaataatccc atccaactac acattaacca caaaatccaa cataataact aataaacaan      60

gaatc                                                                  65


<210>  10
<211>  33
<212>  DNA
<213>  Artificial

<220>
<223>  Oligonucleotide

<400>  10
gttattatgt tggattttgt ggttaatgtg tag                                   33


<210>  11
<211>  89
<212>  DNA
<213>  Artificial

<220>
<223>  Oligonucleotide


<220>
<221>  misc_feature
<222>  (24)..(24)
<223>  3' third region linker

<220>
<221>  misc_feature
<222>  (84)..(84)
<223>  abasic site by Tetrahydrofuran modification (also known as
      dSpacer)

<400>  11
ttatgttgga ttttgtggtt aatgaaataa tcccatccaa ctacgcgtta accgcgaaat      60

ccgacataat aactaataaa caangaatc                                        89


<210>  12
<211>  89
<212>  DNA
<213>  Artificial

<220>
<223>  Oligonucleotide


<220>
<221>  misc_feature
<222>  (24)..(24)
<223>  3' third region linker

<220>
<221>  misc_feature
<222>  (84)..(84)
<223>  abasic site by Tetrahydrofuran modification (also known as
      dSpacer)

<400>  12
ttatgttgga ttttgtggtt aatgaaataa tcccatccaa ctacacatta accacaaaat      60

ccaacataat aactaataaa caangaatc                                        89